(19) Europäisches Patentamt / European Patent Office / Office européen des brevets

(11) EP 3 842 071 A1

(12) **EUROPEAN PATENT APPLICATION**
published in accordance with Art. 153(4) EPC

(43) Date of publication:
30.06.2021 Bulletin 2021/26

(51) Int Cl.:
*A61K 39/395* (2006.01)    *A61P 35/00* (2006.01)

(21) Application number: 19851093.5

(22) Date of filing: 20.08.2019

(86) International application number:
PCT/CN2019/101552

(87) International publication number:
WO 2020/038355 (27.02.2020 Gazette 2020/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 20.08.2018 CN 201810946361

(71) Applicants:
• Jiangsu Hengrui Medicine Co., Ltd.
Lianyungang, Jiangsu 222047 (CN)
• Shanghai Hengrui Pharmaceutical Co., Ltd.
Shanghai 200245 (CN)

(72) Inventors:
• SUN, Xing
Lianyungang, Jiangsu 222047 (CN)
• CAO, Zhuoxiao
Lianyungang, Jiangsu 222047 (CN)
• XU, Zupeng
Lianyungang, Jiangsu 222047 (CN)
• LIAO, Cheng
Lianyungang, Jiangsu 222047 (CN)
• YANG, Changyong
Lianyungang, Jiangsu 222047 (CN)
• ZHANG, Lianshan
Lianyungang, Jiangsu 222047 (CN)

(74) Representative: Sonzogni, Laura Gabriella et al
Dragotti & Associati S.r.l.
Via Nino Bixio, 7
20129 Milano (IT)

(54) **USE OF TIM-3 ANTIBODY IN PREPARATION OF MEDICINES FOR TREATING TUMORS**

(57) Disclosed is use of a TIM-3 antibody in preparation of medicines for treating tumors. Specifically, provided is use of the TIM-3 antibody or an antigen-binding fragment thereof in preparation of medicines for treating non-small cell lung cancer, the TIM-3 antibody containing a heavy chain variable region shown in SEQ ID NO: 33 and a light chain variable region shown in SEQ ID NO: 36. Further, also provided is use of the TIM-3 antibody or the antigen-binding fragment thereof and a PD-1 antibody or an antigen-binding fragment thereof in joint preparation of medicines for treating tumors.

EP 3 842 071 A1

Sequence Listing

<110>  JIANGSU HENGRUI MEDICINE CO., LTD.; SHANGHAI HENGRUI PHARMACEUTICAL
CO., LTD.

<120>  USE OF TIM-3 ANTIBODY IN PREPARATION OF MEDICINES FOR TREATING TUMORS

<130>  719057CPCT

<160>  82

<170>  SIPOSequenceListing 1.0

<210>  1
<211>  430
<212>  PRT
<213>  Artificial Sequence

<220>
<221>  DOMAIN
<222>  (1)..(430)
<223>  Fusion of TIM-3 extracellular domain and hIgG1 Fc


<400>  1
Met Glu Phe Gly Leu Ser Trp Leu Phe Leu Val Ala Ile Leu Lys Gly
1               5                   10                  15
Val Gln Cys Ser Glu Val Glu Tyr Arg Ala Glu Val Gly Gln Asn Ala
            20                  25                  30
Tyr Leu Pro Cys Phe Tyr Thr Pro Ala Ala Pro Gly Asn Leu Val Pro
        35                  40                  45
Val Cys Trp Gly Lys Gly Ala Cys Pro Val Phe Glu Cys Gly Asn Val
        50                  55                  60
Val Leu Arg Thr Asp Glu Arg Asp Val Asn Tyr Trp Thr Ser Arg Tyr
65                  70                  75                  80
Trp Leu Asn Gly Asp Phe Arg Lys Gly Asp Val Ser Leu Thr Ile Glu
            85                  90                  95
Asn Val Thr Leu Ala Asp Ser Gly Ile Tyr Cys Cys Arg Ile Gln Ile
            100                 105                 110
Pro Gly Ile Met Asn Asp Glu Lys Phe Asn Leu Lys Leu Val Ile Lys
        115                 120                 125
Pro Ala Lys Val Thr Pro Ala Pro Thr Arg Gln Arg Asp Phe Thr Ala
        130                 135                 140
Ala Phe Pro Arg Met Leu Thr Thr Arg Gly His Gly Pro Ala Glu Thr
145                 150                 155                 160
Gln Thr Leu Gly Ser Leu Pro Asp Ile Asn Leu Thr Gln Ile Ser Thr
            165                 170                 175
Leu Ala Asn Glu Leu Arg Asp Ser Arg Leu Ala Asn Asp Leu Arg Asp
            180                 185                 190
Ser Gly Ala Thr Ile Arg Glu Pro Lys Ser Ser Asp Lys Thr His Thr
            195                 200                 205
Cys Pro Pro Cys Pro Ala Pro Glu Leu Leu Gly Gly Pro Ser Val Phe
        210                 215                 220
Leu Phe Pro Pro Lys Pro Lys Asp Thr Leu Met Ile Ser Arg Thr Pro
225                 230                 235                 240
Glu Val Thr Cys Val Val Val Asp Val Ser His Glu Asp Pro Glu Val
            245                 250                 255
Lys Phe Asn Trp Tyr Val Asp Gly Val Glu Val His Asn Ala Lys Thr
            260                 265                 270
Lys Pro Arg Glu Glu Gln Tyr Asn Ser Thr Tyr Arg Val Val Ser Val
            275                 280                 285
Leu Thr Val Leu His Gln Asp Trp Leu Asn Gly Lys Glu Tyr Lys Cys
        290                 295                 300

```
Lys Val Ser Asn Lys Ala Leu Pro Ala Pro Ile Glu Lys Thr Ile Ser
305                 310                 315                 320
Lys Ala Lys Gly Gln Pro Arg Glu Pro Gln Val Tyr Thr Leu Pro Pro
            325                 330                 335
Ser Arg Asp Glu Leu Thr Lys Asn Gln Val Ser Leu Thr Cys Leu Val
            340                 345                 350
Lys Gly Phe Tyr Pro Ser Asp Ile Ala Val Glu Trp Glu Ser Asn Gly
            355                 360                 365
Gln Pro Glu Asn Asn Tyr Lys Thr Thr Pro Pro Val Leu Asp Ser Asp
    370                 375                 380
Gly Ser Phe Phe Leu Tyr Ser Lys Leu Thr Val Asp Lys Ser Arg Trp
385                 390                 395                 400
Gln Gln Gly Asn Val Phe Ser Cys Ser Val Met His Glu Ala Leu His
            405                 410                 415
Asn His Tyr Thr Gln Lys Ser Leu Ser Leu Ser Pro Gly Lys
            420                 425                 430
```

```
<210>  2
<211>  215
<212>  PRT
<213>  Artificial Sequence

<220>
<221>  DOMAIN
<222>  (1)..(215)
<223>  TIM-3 extracellular domain with Flag-tag and His-tag


<400>  2
Met Glu Phe Gly Leu Ser Trp Leu Phe Leu Val Ala Ile Leu Lys Gly
1               5                   10                  15
Val Gln Cys Ser Glu Val Glu Tyr Arg Ala Glu Val Gly Gln Asn Ala
            20                  25                  30
Tyr Leu Pro Cys Phe Tyr Thr Pro Ala Ala Pro Gly Asn Leu Val Pro
        35                  40                  45
Val Cys Trp Gly Lys Gly Ala Cys Pro Val Phe Glu Cys Gly Asn Val
        50                  55                  60
Val Leu Arg Thr Asp Glu Arg Asp Val Asn Tyr Trp Thr Ser Arg Tyr
65                  70                  75                  80
Trp Leu Asn Gly Asp Phe Arg Lys Gly Asp Val Ser Leu Thr Ile Glu
                85                  90                  95
Asn Val Thr Leu Ala Asp Ser Gly Ile Tyr Cys Cys Arg Ile Gln Ile
            100                 105                 110
Pro Gly Ile Met Asn Asp Glu Lys Phe Asn Leu Lys Leu Val Ile Lys
        115                 120                 125
Pro Ala Lys Val Thr Pro Ala Pro Thr Arg Gln Arg Asp Phe Thr Ala
    130                 135                 140
Ala Phe Pro Arg Met Leu Thr Thr Arg Gly His Gly Pro Ala Glu Thr
145                 150                 155                 160
Gln Thr Leu Gly Ser Leu Pro Asp Ile Asn Leu Thr Gln Ile Ser Thr
                165                 170                 175
Leu Ala Asn Glu Leu Arg Asp Ser Arg Leu Ala Asn Asp Leu Arg Asp
            180                 185                 190
Ser Gly Ala Thr Ile Arg Gly Ser Ser Asp Tyr Lys Asp Asp Asp Asp
        195                 200                 205
Lys His His His His His
    210                 215


<210>  3
<211>  301
<212>  PRT
<213>  Artificial Sequence
```

```
<220>
<221>    CHAIN
<222>    (1)..(301)
<223>    TIM-3 full-length sequence


<400>    3
Met Phe Ser His Leu Pro Phe Asp Cys Val Leu Leu Leu Leu Leu Leu
1               5                   10                  15
Leu Leu Thr Arg Ser Ser Glu Val Glu Tyr Arg Ala Glu Val Gly Gln
            20                  25                  30
Asn Ala Tyr Leu Pro Cys Phe Tyr Thr Pro Ala Ala Pro Gly Asn Leu
        35                  40                  45
Val Pro Val Cys Trp Gly Lys Gly Ala Cys Pro Val Phe Glu Cys Gly
    50                  55                  60
Asn Val Val Leu Arg Thr Asp Glu Arg Asp Val Asn Tyr Trp Thr Ser
65                  70                  75                  80
Arg Tyr Trp Leu Asn Gly Asp Phe Arg Lys Gly Asp Val Ser Leu Thr
                85                  90                  95
Ile Glu Asn Val Thr Leu Ala Asp Ser Gly Ile Tyr Cys Cys Arg Ile
            100                 105                 110
Gln Ile Pro Gly Ile Met Asn Asp Glu Lys Phe Asn Leu Lys Leu Val
        115                 120                 125
Ile Lys Pro Ala Lys Val Thr Pro Ala Pro Thr Arg Gln Arg Asp Phe
    130                 135                 140
Thr Ala Ala Phe Pro Arg Met Leu Thr Thr Arg Gly His Gly Pro Ala
145                 150                 155                 160
Glu Thr Gln Thr Leu Gly Ser Leu Pro Asp Ile Asn Leu Thr Gln Ile
                165                 170                 175
Ser Thr Leu Ala Asn Glu Leu Arg Asp Ser Arg Leu Ala Asn Asp Leu
            180                 185                 190
Arg Asp Ser Gly Ala Thr Ile Arg Ile Gly Ile Tyr Ile Gly Ala Gly
        195                 200                 205
Ile Cys Ala Gly Leu Ala Leu Ala Leu Ile Phe Gly Ala Leu Ile Phe
    210                 215                 220
Lys Trp Tyr Ser His Ser Lys Glu Lys Ile Gln Asn Leu Ser Leu Ile
225                 230                 235                 240
Ser Leu Ala Asn Leu Pro Pro Ser Gly Leu Ala Asn Ala Val Ala Glu
                245                 250                 255
Gly Ile Arg Ser Glu Glu Asn Ile Tyr Thr Ile Glu Glu Asn Val Tyr
            260                 265                 270
Glu Val Glu Glu Pro Asn Glu Tyr Tyr Cys Tyr Val Ser Ser Arg Gln
        275                 280                 285
Gln Pro Ser Gln Pro Leu Gly Cys Arg Phe Ala Met Pro
    290                 295                 300


<210>    4
<211>    121
<212>    PRT
<213>    Mus musculus


<220>
<221>    DOMAIN
<222>    (1)..(121)
<223>    mAb-1701 antibody heavy chain variable region


<400>    4
Glu Val Gln Leu Gln Gln Ser Gly Pro Glu Leu Val Lys Pro Gly Ala
1               5                   10                  15
Ser Val Lys Ile Ser Cys Lys Ala Ser Gly Tyr Thr Phe Thr Asp Tyr
            20                  25                  30
Tyr Met Asn Trp Val Lys Gln Ser His Gly Lys Ser Leu Glu Trp Ile
```

```
                35                    40                    45
Ala Asp Ile Ile Pro Asn Asn Gly Gly Ser Lys Tyr Asn Gln Lys Phe
    50                    55                    60
Lys Asp Lys Ala Thr Leu Thr Val Asp Lys Ser Ser Thr Ala Tyr
65                    70                    75                    80
Met Glu Leu Arg Ser Leu Thr Ser Glu Asp Ser Ala Val Tyr Tyr Cys
                85                    90                    95
Ala Thr Trp Gly Tyr Gly Ser Ser Tyr Arg Trp Phe Asp Tyr Trp Gly
               100                   105                   110
Gln Gly Thr Leu Val Ser Val Ser Ala
               115                   120
```

```
<210>   5
<211>   107
<212>   PRT
<213>   Mus musculus

<220>
<221>   DOMAIN
<222>   (1)..(107)
<223>   mAb-1701 antibody light chain variable region


<400>   5
Asp Ile Gln Met Thr Gln Ser Pro Ala Ser Gln Ser Ala Ser Leu Gly
1                5                    10                   15
Glu Ser Val Thr Ile Thr Cys Leu Ala Ser Gln Pro Ile Gly Ile Trp
                20                   25                   30
Leu Ala Trp Tyr Gln Gln Lys Pro Gly Lys Ser Pro Gln Leu Leu Ile
        35                    40                    45
Tyr Ala Ala Thr Ser Leu Ala Asp Gly Val Pro Ser Arg Phe Ser Gly
    50                    55                    60
Ser Gly Ser Gly Thr Lys Phe Ser Phe Lys Ile Ser Ser Leu Gln Ala
65                    70                    75                    80
Glu Asp Phe Val Ser Tyr Tyr Cys Gln Gln Leu Tyr Ser Ser Pro Trp
                85                    90                    95
Thr Phe Gly Gly Gly Thr Lys Leu Glu Ile Lys
               100                   105
```

```
<210>   6
<211>   122
<212>   PRT
<213>   Mus musculus

<220>
<221>   DOMAIN
<222>   (1)..(122)
<223>   mAb-1799 antibody heavy chain variable region


<400>   6
Glu Val Lys Leu Val Glu Ser Glu Gly Gly Leu Val Gln Pro Gly Ser
1                5                    10                   15
Ser Met Lys Leu Ser Cys Thr Ala Ser Gly Phe Thr Phe Ser Asp Tyr
                20                   25                   30
Tyr Met Ala Trp Val Arg Gln Val Pro Glu Lys Gly Leu Glu Trp Val
        35                    40                    45
Ala Asn Ile Asn Tyr Asp Gly Ser Ser Thr Tyr Tyr Leu Asp Ser Leu
    50                    55                    60
Lys Ser Arg Phe Ile Ile Ser Arg Asp Asn Ala Lys Asn Ile Leu Tyr
65                    70                    75                    80
Leu Gln Met Asn Ser Leu Lys Ser Asp Asp Thr Ala Thr Tyr Tyr Cys
                85                    90                    95
```

```
Ala Arg Asp Val Gly Tyr Tyr Gly Gly Asn Tyr Gly Phe Ala Tyr Trp
            100                 105                 110
Gly Gln Gly Thr Leu Val Thr Val Ser Ala
            115                 120


<210>  7
<211>  107
<212>  PRT
<213>  Mus musculus


<220>
<221>  DOMAIN
<222>  (1)..(107)
<223>  mAb-1799 antibody light chain variable region



<400>  7
Asp Ile Gln Met Thr Gln Ser Pro Ala Ser Leu Ser Ala Ser Val Gly
1               5                   10                  15
Glu Thr Val Thr Ile Thr Cys Arg Ala Ser Asp Asn Ile Tyr Ser Tyr
            20                  25                  30
Leu Ala Trp Tyr Gln Gln Lys Gln Gly Lys Ser Pro Gln Leu Leu Val
            35                  40                  45
Tyr Asn Ala Lys Thr Leu Ala Glu Gly Val Pro Ser Arg Phe Ser Gly
        50                  55                  60
Ser Gly Ser Gly Thr Gln Phe Ser Leu Lys Ile Asn Ser Leu Gln Pro
65                  70                  75                  80
Glu Asp Phe Gly Ser Tyr Tyr Cys Gln Gln His Tyr Gly Ser Pro Leu
                85                  90                  95
Thr Phe Gly Ala Gly Thr Lys Leu Glu Leu Lys
            100                 105


<210>  8
<211>  5
<212>  PRT
<213>  Mus musculus


<220>
<221>  DOMAIN
<222>  (1)..(5)
<223>  mAb-1701 antibody heavy chain HCDR1


<400>  8
Asp Tyr Tyr Met Asn
1               5


<210>  9
<211>  17
<212>  PRT
<213>  Mus musculus


<220>
<221>  DOMAIN
<222>  (1)..(17)
<223>  mAb-1701 antibody heavy chain HCDR2


<400>  9
Asp Ile Ile Pro Asn Asn Gly Gly Ser Lys Tyr Asn Gln Lys Phe Lys
1               5                   10                  15
Asp
```

```
<210>  10
<211>  12
<212>  PRT
<213>  Mus musculus

<220>
<221>  DOMAIN
<222>  (1)..(12)
<223>  mAb-1701 antibody heavy chain HCDR3


<400>  10
Trp Gly Tyr Gly Ser Ser Tyr Arg Trp Phe Asp Tyr
1               5                   10

<210>  11
<211>  11
<212>  PRT
<213>  Mus musculus

<220>
<221>  DOMAIN
<222>  (1)..(11)
<223>  mAb-1701 antibody light chain LCDR1


<400>  11
Leu Ala Ser Gln Pro Ile Gly Ile Trp Leu Ala
1               5                   10

<210>  12
<211>  7
<212>  PRT
<213>  Mus musculus

<220>
<221>  DOMAIN
<222>  (1)..(7)
<223>  mAb-1701 antibody light chain LCDR2


<400>  12
Ala Ala Thr Ser Leu Ala Asp
1               5

<210>  13
<211>  9
<212>  PRT
<213>  Mus musculus

<220>
<221>  DOMAIN
<222>  (1)..(9)
<223>  mAb-1701 antibody light chain LCDR3


<400>  13
Gln Gln Leu Tyr Ser Ser Pro Trp Thr
1               5

<210>  14
<211>  5
```

```
<212> PRT
<213> Mus musculus

<220>
<221> DOMAIN
<222> (1)..(5)
<223> mAb-1799 antibody heavy chain HCDR1


<400> 14
Asp Tyr Tyr Met Ala
1               5

<210> 15
<211> 17
<212> PRT
<213> Mus musculus

<220>
<221> DOMAIN
<222> (1)..(17)
<223> mAb-1799 antibody heavy chain HCDR2


<400> 15
Asn Ile Asn Tyr Asp Gly Ser Ser Thr Tyr Tyr Leu Asp Ser Leu Lys
1               5                   10                  15
Ser


<210> 16
<211> 13
<212> PRT
<213> Mus musculus

<220>
<221> DOMAIN
<222> (1)..(13)
<223> mAb-1799 antibody heavy chain HCDR3


<400> 16
Asp Val Gly Tyr Tyr Gly Gly Asn Tyr Gly Phe Ala Tyr
1               5                   10

<210> 17
<211> 11
<212> PRT
<213> Mus musculus

<220>
<221> DOMAIN
<222> (1)..(11)
<223> mAb-1799 antibody light chain LCDR1


<400> 17
Arg Ala Ser Asp Asn Ile Tyr Ser Tyr Leu Ala
1               5                   10

<210> 18
<211> 7
<212> PRT
```

<213> Mus musculus

<220>
<221> DOMAIN
<222> (1)..(7)
<223> mAb-1799 antibody light chain LCDR2

<400> 18
Asn Ala Lys Thr Leu Ala Glu
1               5

<210> 19
<211> 9
<212> PRT
<213> Mus musculus

<220>
<221> DOMAIN
<222> (1)..(9)
<223> mAb-1799 antibody light chain LCDR3

<400> 19
Gln Gln His Tyr Gly Ser Pro Leu Thr
1               5

<210> 20
<211> 121
<212> PRT
<213> Artificial Sequence

<220>
<221> DOMAIN
<222> (1)..(121)
<223> murine mAb-1701 humanized heavy chain variable region

<400> 20
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
                100                 105                 110
Gln Gly Thr Leu Val Thr Val Ser Ser
            115                 120

<210> 21
<211> 107
<212> PRT
<213> Artificial Sequence

<220>
<221> DOMAIN

33

```
<222>    (1)..(107)
<223>    murine mAb-1701 humanized light chain variable region


<400>    21
Asp Ile Gln Met Thr Gln Ser Pro Ser Ser Leu Ser Ala Ser Val Gly
1                   5                   10                  15
Asp Arg Val Thr Ile Thr Cys Leu Ala Ser Gln Pro Ile Gly Ile Trp
            20                  25                  30
Leu Ala Trp Tyr Gln Gln Lys Pro Gly Lys Ala Pro Lys Leu Leu Ile
        35                  40                  45
Tyr Ala Ala Thr Ser Leu Ala Asp Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Leu Tyr Ser Ser Pro Trp
                85                  90                  95
Thr Phe Gly Gly Gly Thr Lys Val Glu Ile Lys
            100                 105


<210>    22
<211>    121
<212>    PRT
<213>    Artificial Sequence

<220>
<221>    DOMAIN
<222>    (1)..(121)
<223>    h1701_VH.1


<400>    22
Gln Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
            100                 105                 110
Gln Gly Thr Leu Val Thr Val Ser Ser
            115                 120


<210>    23
<211>    121
<212>    PRT
<213>    Artificial Sequence

<220>
<221>    DOMAIN
<222>    (1)..(121)
<223>    h1701h1701_VH.1A


<400>    23
Gln Val Gln Leu Val Gln Ser Gly Ala Glu Val Lys Lys Pro Gly Ala
1                   5                   10                  15
```

34

```
Ser Val Lys Val Ser Cys Lys Ala Ser Gly Tyr Thr Phe Thr Asp Tyr
        20                  25                  30
Tyr Met Asn Trp Val Arg Gln Ala Pro Gly Gln Gly Leu Glu Trp Ile
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
            100                 105                 110
Gln Gly Thr Leu Val Thr Val Ser Ser
            115                 120
```

```
<210>    24
<211>    121
<212>    PRT
<213>    Artificial Sequence

<220>
<221>    DOMAIN
<222>    (1)..(121)
<223>    h1701_VH.1B


<400>    24
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
        20                  25                  30
Tyr Met Asn Trp Val Arg Gln Ala Pro Gly Gln Gly Leu Glu Trp Met
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
            115                 120
```

```
<210>    25
<211>    121
<212>    PRT
<213>    Artificial Sequence

<220>
<221>    DOMAIN
<222>    (1)..(121)
<223>    h1701_VH.1C


<400>    25
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
        20                  25                  30
Tyr Met Asn Trp Val Arg Gln Ala Pro Gly Gln Gly Leu Glu Trp Ile
        35                  40                  45
Gly Asp Ile Ile Pro Asn Asn Gly Gly Ser Lys Tyr Asn Gln Lys Phe
```

```
                50                      55                      60
       Lys Asp Arg Val Thr Met Thr Thr Asp Thr Ser Thr Ser Thr Ala Tyr
       65                      70                      75                      80
       Met Glu Leu Arg Ser Leu Arg Ser Asp Asp Thr Ala Val Tyr Tyr Cys
                               85                      90                      95
       Ala Thr Trp Gly Tyr Gly Ser Ser Tyr Arg Trp Phe Asp Tyr Trp Gly
                       100                     105                     110
       Gln Gly Thr Leu Val Thr Val Ser Ser
                       115                     120


       <210>   26
       <211>   121
       <212>   PRT
       <213>   Artificial Sequence

       <220>
       <221>   DOMAIN
       <222>   (1)..(121)
       <223>   h1701_VH.1D


       <400>   26
       Gln Val Gln Leu Val Gln Ser Gly Ala Glu Val Lys Lys Pro Gly Ala
       1                       5                       10                      15
       Ser Val Lys Val Ser Cys Lys Ala Ser Gly Tyr Thr Phe Thr Asp Tyr
                       20                      25                      30
       Tyr Met Asn Trp Val Lys Gln Ala Pro Gly Gln Gly Leu Glu Trp Ile
                       35                      40                      45
       Gly Asp Ile Ile Pro Asn Asn Gly Gly Ser Lys Tyr Asn Gln Lys Phe
                50                      55                      60
       Lys Asp Arg Val Thr Met Thr Thr Asp Thr Ser Thr Ser Thr Ala Tyr
       65                      70                      75                      80
       Met Glu Leu Arg Ser Leu Arg Ser Glu Asp Thr Ala Val Tyr Tyr Cys
                               85                      90                      95
       Ala Thr Trp Gly Tyr Gly Ser Ser Tyr Arg Trp Phe Asp Tyr Trp Gly
                       100                     105                     110
       Gln Gly Thr Leu Val Thr Val Ser Ser
                       115                     120


       <210>   27
       <211>   121
       <212>   PRT
       <213>   Artificial Sequence

       <220>
       <221>   DOMAIN
       <222>   (1)..(121)
       <223>   h1701_VH.1E


       <400>   27
       Gln Val Gln Leu Val Gln Ser Gly Ala Glu Val Lys Lys Pro Gly Ala
       1                       5                       10                      15
       Ser Val Lys Val Ser Cys Lys Ala Ser Gly Tyr Thr Phe Thr Asp Tyr
                       20                      25                      30
       Tyr Met Asn Trp Val Arg Gln Ala Pro Gly Gln Gly Leu Glu Trp Ile
                       35                      40                      45
       Ala Asp Ile Ile Pro Asn Asn Gly Gly Ser Lys Tyr Asn Gln Lys Phe
                50                      55                      60
       Lys Asp Arg Ala Thr Leu Thr Thr Asp Thr Ser Thr Ser Thr Ala Tyr
       65                      70                      75                      80
       Met Glu Leu Arg Ser Leu Arg Ser Asp Asp Thr Ala Val Tyr Tyr Cys
                               85                      90                      95
```

```
        Ala Thr Trp Gly Tyr Gly Ser Ser Tyr Arg Trp Phe Asp Tyr Trp Gly
                    100                 105             110
        Gln Gly Thr Leu Val Thr Val Ser Ser
                    115                 120


        <210>   28
        <211>   121
        <212>   PRT
        <213>   Artificial Sequence


        <220>
        <221>   DOMAIN
        <222>   (1)..(121)
        <223>   h1701_VH.1F


        <400>   28
        Gln Val Gln Leu Val Gln Ser Gly Ala Glu Val Lys Lys Pro Gly Ala
        1                   5                   10                  15
        Ser Val Lys Val Ser Cys Lys Ala Ser Gly Tyr Thr Phe Thr Asp Tyr
                    20                  25                  30
        Tyr Met Asn Trp Val Lys Gln Ala Pro Gly Gln Gly Leu Glu Trp Ile
                    35                  40                  45
        Ala Asp Ile Ile Pro Asn Asn Gly Gly Ser Lys Tyr Asn Gln Lys Phe
                    50                  55                  60
        Lys Asp Arg Ala Thr Leu Thr Thr Asp Thr Ser Thr Ser Thr Ala Tyr
        65                  70                  75                  80
        Met Glu Leu Arg Ser Leu Arg Ser Glu Asp Thr Ala Val Tyr Tyr Cys
                    85                  90                  95
        Ala Thr Trp Gly Tyr Gly Ser Ser Tyr Arg Trp Phe Asp Tyr Trp Gly
                    100                 105             110
        Gln Gly Thr Leu Val Thr Val Ser Ser
                    115                 120


        <210>   29
        <211>   107
        <212>   PRT
        <213>   Artificial Sequence


        <220>
        <221>   DOMAIN
        <222>   (1)..(107)
        <223>   h1701_VL.1


        <400>   29
        Asp Ile Gln Met Thr Gln Ser Pro Ser Ser Leu Ser Ala Ser Val Gly
        1                   5                   10                  15
        Asp Arg Val Thr Ile Thr Cys Leu Ala Ser Gln Pro Ile Gly Ile Trp
                    20                  25                  30
        Leu Ala Trp Tyr Gln Gln Lys Pro Gly Lys Ala Pro Lys Leu Leu Ile
                    35                  40                  45
        Tyr Ala Ala Thr Ser Leu Ala Asp Gly Val Pro Ser Arg Phe Ser Gly
                    50                  55                  60
        Ser Gly Ser Gly Thr Asp Phe Thr Phe Thr Ile Ser Ser Leu Gln Pro
        65                  70                  75                  80
        Glu Asp Ile Ala Thr Tyr Tyr Cys Gln Gln Leu Tyr Ser Ser Pro Trp
                    85                  90                  95
        Thr Phe Gly Gly Gly Thr Lys Val Glu Ile Lys
                    100                 105


        <210>   30
        <211>   107
```

<212>    PRT
<213>    Artificial Sequence

<220>
<221>    DOMAIN
<222>    (1)..(107)
<223>    h1701_VL.1A


<400>    30
Asp Ile Gln Met Thr Gln Ser Pro Ser Ser Leu Ser Ala Ser Val Gly
1                   5                   10                  15
Asp Arg Val Thr Ile Thr Cys Leu Ala Ser Gln Pro Ile Gly Ile Trp
                20                  25                  30
Leu Ala Trp Tyr Gln Gln Lys Pro Gly Lys Ser Pro Lys Leu Leu Ile
            35                  40                  45
Tyr Ala Ala Thr Ser Leu Ala Asp Gly Val Pro Ser Arg Phe Ser Gly
        50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Leu Tyr Ser Ser Pro Trp
                85                  90                  95
Thr Phe Gly Gly Gly Thr Lys Val Glu Ile Lys
            100                 105

<210>    31
<211>    122
<212>    PRT
<213>    Artificial Sequence

<220>
<221>    DOMAIN
<222>    (1)..(122)
<223>    h1799VH-CDR graft


<400>    31
Glu Val Gln Leu Val Glu Ser Gly Gly Gly Leu Val Gln Pro Gly Gly
1                   5                   10                  15
Ser Leu Arg Leu Ser Cys Ala Ala Ser Gly Phe Thr Phe Ser Asp Tyr
                20                  25                  30
Tyr Met Ala Trp Val Arg Gln Ala Pro Gly Lys Gly Leu Glu Trp Val
            35                  40                  45
Ala Asn Ile Asn Tyr Asp Gly Ser Ser Thr Tyr Tyr Leu Asp Ser Leu
        50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
            115                 120

<210>    32
<211>    107
<212>    PRT
<213>    Artificial Sequence

<220>
<221>    DOMAIN
<222>    (1)..(107)
<223>    h1799VL-CDR graft

<400> 32

```
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Lys Leu Leu Ile
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105
```

<210> 33
<211> 122
<212> PRT
<213> Artificial Sequence

<220>
<221> DOMAIN
<222> (1)..(122)
<223> h1799_VH.1


<400> 33

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
        35                  40                  45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
            115                 120
```

<210> 34
<211> 122
<212> PRT
<213> Artificial Sequence

<220>
<221> DOMAIN
<222> (1)..(122)
<223> h1799_VH.1A


<400> 34

```
Glu Val Lys Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
```

```
Tyr Met Ala Trp Val Arg Gln Ala Pro Gly Lys Gly Leu Glu Trp Val
    35                      40              45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
        115                 120
```

```
<210>   35
<211>   122
<212>   PRT
<213>   Artificial Sequence

<220>
<221>   DOMAIN
<222>   (1)..(122)
<223>   h1799_VH.1B
```

```
<400>   35
Glu Val Lys Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
    35                      40              45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Lys Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
        115                 120
```

```
<210>   36
<211>   107
<212>   PRT
<213>   Artificial Sequence

<220>
<221>   DOMAIN
<222>   (1)..(107)
<223>   h1799_VL.1
```

```
<400>   36
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Lys Leu Leu Ile
    35                      40              45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
```

```
                              65                      70                      75                      80
                              Glu Asp Phe Ala Thr Tyr Tyr Cys Gln Gln His Tyr Gly Ser Pro Leu
                                              85                      90                      95
                              Thr Phe Gly Gln Gly Thr Lys Leu Glu Ile Lys
                                          100                     105


                              <210>   37
                              <211>   107
                              <212>   PRT
                              <213>   Artificial Sequence

                              <220>
                              <221>   DOMAIN
                              <222>   (1)..(107)
                              <223>   h1799_VL.1A


                              <400>   37
                              Asp Ile Gln Met Thr Gln Ser Pro Ser Ser Leu Ser Ala Ser Val Gly
                              1                   5                   10                      15
                              Asp Arg Val Thr Ile Thr Cys Arg Ala Ser Asp Asn Ile Tyr Ser Tyr
                                              20                      25                      30
                              Leu Ala Trp Tyr Gln Gln Lys Pro Gly Lys Ala Pro Lys Leu Leu Val
                                          35                      40                      45
                              Tyr Asn Ala Lys Thr Leu Ala Glu Gly Val Pro Ser Arg Phe Ser Gly
                                      50                      55                      60
                              Ser Gly Ser Gly Thr Asp Phe Thr Leu Thr Ile Ser Ser Leu Gln Pro
                              65                      70                      75                      80
                              Glu Asp Phe Ala Thr Tyr Tyr Cys Gln Gln His Tyr Gly Ser Pro Leu
                                              85                      90                      95
                              Thr Phe Gly Gln Gly Thr Lys Leu Glu Ile Lys
                                          100                     105


                              <210>   38
                              <211>   107
                              <212>   PRT
                              <213>   Artificial Sequence

                              <220>
                              <221>   DOMAIN
                              <222>   (1)..(107)
                              <223>   h1799_VL.1B


                              <400>   38
                              Asp Ile Gln Met Thr Gln Ser Pro Ser Ser Leu Ser Ala Ser Val Gly
                              1                   5                   10                      15
                              Asp Arg Val Thr Ile Thr Cys Arg Ala Ser Asp Asn Ile Tyr Ser Tyr
                                              20                      25                      30
                              Leu Ala Trp Tyr Gln Gln Lys Pro Gly Lys Ala Pro Gln Leu Leu Val
                                          35                      40                      45
                              Tyr Asn Ala Lys Thr Leu Ala Glu Gly Val Pro Ser Arg Phe Ser Gly
                                      50                      55                      60
                              Ser Gly Ser Gly Thr Asp Phe Thr Leu Thr Ile Ser Ser Leu Gln Pro
                              65                      70                      75                      80
                              Glu Asp Phe Ala Thr Tyr Tyr Cys Gln Gln His Tyr Gly Ser Pro Leu
                                              85                      90                      95
                              Thr Phe Gly Gln Gly Thr Lys Leu Glu Ile Lys
                                          100                     105


                              <210>   39
                              <211>   107
                              <212>   PRT
```

<210> Artificial Sequence

<220>
<221> DOMAIN
<222> (1)..(107)
<223> h1799_VL.1C


<400> 39
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ser Pro Gln Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105


<210> 40
<211> 107
<212> PRT
<213> Artificial Sequence


<220>
<221> DOMAIN
<222> (1)..(107)
<223> h1799_VL.1D


<400> 40
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ser Pro Gln Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Ser Tyr Tyr Cys Gln Gln His Tyr Gly Ser Pro Leu
                85                  90                  95
Thr Phe Gly Gln Gly Thr Lys Leu Glu Ile Lys
            100                 105


<210> 41
<211> 327
<212> PRT
<213> Artificial Sequence


<220>
<221> DOMAIN
<222> (1)..(327)
<223> heavy chain constant region


<400> 41


42

2

```
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
            195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
            245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325
```

```
<210>  42
<211>  107
<212>  PRT
<213>  Artificial Sequence

<220>
<221>  DOMAIN
<222>  (1)..(107)
<223>  light chain constant region


<400>  42
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
```

```
Thr Tyr Ser Leu Ser Ser Thr Leu Thr Leu Ser Lys Ala Asp Tyr Glu
65                  70              75                      80
Lys His Lys Val Tyr Ala Cys Glu Val Thr His Gln Gly Leu Ser Ser
                85                  90                      95
Pro Val Thr Lys Ser Phe Asn Arg Gly Glu Cys
            100                 105


<210>   43
<211>   17
<212>   PRT
<213>   Artificial Sequence

<220>
<221>   DOMAIN
<222>   (5)..(5)
<223>   Xaa may be Asn, Leu, Val, Met or Glu


<220>
<221>   DOMAIN
<222>   (6)..(6)
<223>   Xaa may be Asn, Glu, Met, His, Lys, Leu, Ala or Val


<220>
<221>   DOMAIN
<222>   (7)..(7)
<223>   Xaa may be Gly or Ala


<400>   43
Asp Ile Ile Pro Xaa Xaa Xaa Gly Ser Lys Tyr Asn Gln Lys Phe Lys
1               5                   10                  15
Asp


<210>   44
<211>   121
<212>   PRT
<213>   Artificial Sequence

<220>
<221>   DOMAIN
<222>   (54)..(54)
<223>   Xaa may be Asn, Leu, Val, Met or Glu


<220>
<221>   DOMAIN
<222>   (55)..(55)
<223>   Xaa may be Asn, Glu, Met, His, Lys, Leu, Ala or Val


<220>
<221>   DOMAIN
<222>   (56)..(56)
<223>   Xaa may be Gly or Ala


<400>   44
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
```

```
                 20                      25                      30
      Tyr Met Asn Trp Val Arg Gln Ala Pro Gly Gln Gly Leu Glu Trp Met
              35                      40                      45
      Gly Asp Ile Ile Pro Xaa Xaa Xaa Gly Ser Lys Tyr Asn Gln Lys Phe
              50                      55                      60
      Lys Asp Arg Val Thr Met Thr Thr Asp Thr Ser Thr Ser Thr Ala Tyr
      65                      70                      75                      80
      Met Glu Leu Arg Ser Leu Arg Ser Asp Asp Thr Ala Val Tyr Tyr Cys
                      85                      90                      95
      Ala Arg Trp Gly Tyr Gly Ser Ser Tyr Arg Trp Phe Asp Tyr Trp Gly
                      100                     105                     110
      Gln Gly Thr Leu Val Thr Val Ser Ser
              115                     120
```

<210> 45
<211> 121
<212> PRT
<213> Artificial Sequence

<220>
<221> DOMAIN
<222> (54)..(54)
<223> Xaa may be Asn, Leu, Val, Met or Glu

<220>
<221> DOMAIN
<222> (55)..(55)
<223> Xaa may be Asn, Glu, Met, His, Lys, Leu, Ala or Val

<220>
<221> DOMAIN
<222> (56)..(56)
<223> Xaa may be Gly or Ala

<400> 45

```
      Gln Val Gln Leu Val Gln Ser Gly Ala Glu Val Lys Lys Pro Gly Ala
      1               5                       10                      15
      Ser Val Lys Val Ser Cys Lys Ala Ser Gly Tyr Thr Phe Thr Asp Tyr
                      20                      25                      30
      Tyr Met Asn Trp Val Arg Gln Ala Pro Gly Gln Gly Leu Glu Trp Ile
              35                      40                      45
      Gly Asp Ile Ile Pro Xaa Xaa Xaa Gly Ser Lys Tyr Asn Gln Lys Phe
              50                      55                      60
      Lys Asp Arg Val Thr Met Thr Thr Asp Thr Ser Thr Ser Thr Ala Tyr
      65                      70                      75                      80
      Met Glu Leu Arg Ser Leu Arg Ser Asp Asp Thr Ala Val Tyr Tyr Cys
                      85                      90                      95
      Ala Arg Trp Gly Tyr Gly Ser Ser Tyr Arg Trp Phe Asp Tyr Trp Gly
                      100                     105                     110
      Gln Gly Thr Leu Val Thr Val Ser Ser
              115                     120
```

<210> 46
<211> 121
<212> PRT
<213> Artificial Sequence

<220>
<221> DOMAIN
<222> (54)..(54)

<223> Xaa may be Asn, Leu, Val, Met or Glu


<220>
<221> DOMAIN
<222> (55)..(55)
<223> Xaa may be Asn, Glu, Met, His, Lys, Leu, Ala or Val


<220>
<221> DOMAIN
<222> (56)..(56)
<223> Xaa may be Gly or Ala


<400> 46
```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Xaa Xaa Xaa Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
            115                 120
```

<210> 47
<211> 121
<212> PRT
<213> Artificial Sequence


<220>
<221> DOMAIN
<222> (54)..(54)
<223> Xaa may be Asn, Leu, Val, Met or Glu


<220>
<221> DOMAIN
<222> (55)..(55)
<223> Xaa may be Asn, Glu, Met, His, Lys, Leu, Ala or Val


<220>
<221> DOMAIN
<222> (56)..(56)
<223> Xaa may be Gly or Ala


<400> 47
```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
```

```
Gly Asp Ile Ile Pro Xaa Xaa Xaa Gly Ser Lys Tyr Asn Gln Lys Phe
    50              55                  60
Lys Asp Arg Val Thr Met Thr Thr Asp Thr Ser Thr Ser Thr Ala Tyr
65              70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
            115                 120
```

```
<210>   48
<211>   121
<212>   PRT
<213>   Artificial Sequence

<220>
<221>   DOMAIN
<222>   (54)..(54)
<223>   Xaa may be Asn, Leu, Val, Met or Glu


<220>
<221>   DOMAIN
<222>   (55)..(55)
<223>   Xaa may be Asn, Glu, Met, His, Lys, Leu, Ala or Val


<220>
<221>   DOMAIN
<222>   (56)..(56)
<223>   Xaa may be Gly or Ala


<400>   48
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Lys Gln Ala Pro Gly Gln Gly Leu Glu Trp Ile
        35                  40                  45
Gly Asp Ile Ile Pro Xaa Xaa Xaa Gly Ser Lys Tyr Asn Gln Lys Phe
    50              55                  60
Lys Asp Arg Val Thr Met Thr Thr Asp Thr Ser Thr Ser Thr Ala Tyr
65              70                  75                  80
Met Glu Leu Arg Ser Leu Arg Ser Glu Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
            115                 120
```

```
<210>   49
<211>   121
<212>   PRT
<213>   Artificial Sequence

<220>
<221>   DOMAIN
<222>   (54)..(54)
<223>   Xaa may be Asn, Leu, Val, Met or Glu
```

```
<220>
<221>  DOMAIN
<222>  (55)..(55)
<223>  Xaa may be Asn, Glu, Met, His, Lys, Leu, Ala or Val


<220>
<221>  DOMAIN
<222>  (56)..(56)
<223>  Xaa may be Gly or Ala


<400>  49
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Ala Asp Ile Ile Pro Xaa Xaa Xaa Gly Ser Lys Tyr Asn Gln Lys Phe
    50                  55                  60
Lys Asp Arg Ala Thr Leu Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120


<210>  50
<211>  121
<212>  PRT
<213>  Artificial Sequence


<220>
<221>  DOMAIN
<222>  (54)..(54)
<223>  Xaa may be Asn, Leu, Val, Met or Glu


<220>
<221>  DOMAIN
<222>  (55)..(55)
<223>  Xaa may be Asn, Glu, Met, His, Lys, Leu, Ala or Val


<220>
<221>  DOMAIN
<222>  (56)..(56)
<223>  Xaa may be Gly or Ala


<400>  50
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Lys Gln Ala Pro Gly Gln Gly Leu Glu Trp Ile
        35                  40                  45
Ala Asp Ile Ile Pro Xaa Xaa Xaa Gly Ser Lys Tyr Asn Gln Lys Phe
    50                  55                  60
Lys Asp Arg Ala Thr Leu Thr Thr Asp Thr Ser Thr Ser Thr Ala Tyr
```

Met Glu Leu Arg Ser Leu Arg Ser Glu Asp Thr Ala Val Tyr Tyr Cys
65                  70                  75                  80

Ala Thr Trp Gly Tyr Gly Ser Ser Tyr Arg Trp Phe Asp Tyr Trp Gly
                85                  90                  95

Gln Gly Thr Leu Val Thr Val Ser Ser
        115                 120


<210> 51
<211> 121
<212> PRT
<213> Artificial Sequence

<220>
<221> DOMAIN
<222> (1)..(121)
<223> h1701-009NLG heavy chain variable region


<400> 51
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Met Asn Trp Val Arg Gln Ala Pro Gly Gln Gly Leu Glu Trp Met
        35                  40                  45

Gly Asp Ile Ile Pro Asn Leu Gly Gly Ser Lys Tyr Asn Gln Lys Phe
        50                  55                  60

Lys Asp Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Thr Trp Gly Tyr Gly Ser Ser Tyr Arg Trp Phe Asp Tyr Trp Gly
                100                 105                 110

Gln Gly Thr Leu Val Thr Val Ser Ser
        115                 120


<210> 52
<211> 121
<212> PRT
<213> Artificial Sequence

<220>
<221> DOMAIN
<222> (1)..(121)
<223> h1701-009NVG heavy chain variable region


<400> 52
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Met Asn Trp Val Arg Gln Ala Pro Gly Gln Gly Leu Glu Trp Met
        35                  40                  45

Gly Asp Ile Ile Pro Asn Val Gly Gly Ser Lys Tyr Asn Gln Lys Phe
        50                  55                  60

Lys Asp Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Thr Trp Gly Tyr Gly Ser Ser Tyr Arg Trp Phe Asp Tyr Trp Gly
                100                 105                 110

Gln Gly Thr Leu Val Thr Val Ser Ser
        115                     120

<210>  53
<211>  121
<212>  PRT
<213>  Artificial Sequence

<220>
<221>  DOMAIN
<222>  (1)..(121)
<223>  h1701-009NNA heavy chain variable region

<400>  53
Gln Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                     120

<210>  54
<211>  121
<212>  PRT
<213>  Artificial Sequence

<220>
<221>  DOMAIN
<222>  (1)..(121)
<223>  h1701-009NMA heavy chain variable region

<400>  54
Gln Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Met Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                     120

<210>  55
<211>  121

<212> PRT
<213> Artificial Sequence

<220>
<221> DOMAIN
<222> (1)..(121)
<223> h1701-009NEA heavy chain variable region


<400> 55
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Glu Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120


<210> 56
<211> 121
<212> PRT
<213> Artificial Sequence

<220>
<221> DOMAIN
<222> (1)..(121)
<223> h1701-009NHA heavy chain variable region


<400> 56
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn His Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210> 57
<211> 121
<212> PRT
<213> Artificial Sequence

<220>
<221> DOMAIN

<222> (1)..(121)
<223> h1701-009NMG heavy chain variable region

<400> 57

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Met Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
            115                 120
```

<210> 58
<211> 121
<212> PRT
<213> Artificial Sequence

<220>
<221> DOMAIN
<222> (1)..(121)
<223> h1701-009NEG heavy chain variable region

<400> 58

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Glu Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
            115                 120
```

<210> 59
<211> 121
<212> PRT
<213> Artificial Sequence

<220>
<221> DOMAIN
<222> (1)..(121)
<223> h1701-009NKG heavy chain variable region

<400> 59

```
Gln Val Gln Leu Val Gln Ser Gly Ala Glu Val Lys Lys Pro Gly Ala
1               5                   10              15
Ser Val Lys Val Ser Cys Lys Ala Ser Gly Tyr Thr Phe Thr Asp Tyr
            20              25              30
Tyr Met Asn Trp Val Arg Gln Ala Pro Gly Gln Gly Leu Glu Trp Met
        35                  40              45
Gly Asp Ile Ile Pro Asn Lys Gly Gly Ser Lys Tyr Asn Gln Lys Phe
    50                  55              60
Lys Asp Arg Val Thr Met Thr Thr Asp Thr Ser Thr Ser Thr Ala Tyr
65              70              75              80
Met Glu Leu Arg Ser Leu Arg Ser Asp Asp Thr Ala Val Tyr Tyr Cys
            85                  90                  95
Ala Thr Trp Gly Tyr Gly Ser Ser Tyr Arg Trp Phe Asp Tyr Trp Gly
            100             105             110
Gln Gly Thr Leu Val Thr Val Ser Ser
            115             120
```

```
<210>   60
<211>   121
<212>   PRT
<213>   Artificial Sequence

<220>
<221>   DOMAIN
<222>   (1)..(121)
<223>   h1701-009NAG heavy chain variable region


<400>   60
Gln Val Gln Leu Val Gln Ser Gly Ala Glu Val Lys Lys Pro Gly Ala
1               5                   10              15
Ser Val Lys Val Ser Cys Lys Ala Ser Gly Tyr Thr Phe Thr Asp Tyr
            20              25              30
Tyr Met Asn Trp Val Arg Gln Ala Pro Gly Gln Gly Leu Glu Trp Met
        35                  40              45
Gly Asp Ile Ile Pro Asn Ala Gly Gly Ser Lys Tyr Asn Gln Lys Phe
    50                  55              60
Lys Asp Arg Val Thr Met Thr Thr Asp Thr Ser Thr Ser Thr Ala Tyr
65              70              75              80
Met Glu Leu Arg Ser Leu Arg Ser Asp Asp Thr Ala Val Tyr Tyr Cys
            85                  90                  95
Ala Thr Trp Gly Tyr Gly Ser Ser Tyr Arg Trp Phe Asp Tyr Trp Gly
            100             105             110
Gln Gly Thr Leu Val Thr Val Ser Ser
            115             120
```

```
<210>   61
<211>   121
<212>   PRT
<213>   Artificial Sequence

<220>
<221>   DOMAIN
<222>   (1)..(121)
<223>   h1701-009NHG heavy chain variable region


<400>   61
Gln Val Gln Leu Val Gln Ser Gly Ala Glu Val Lys Lys Pro Gly Ala
1               5                   10              15
Ser Val Lys Val Ser Cys Lys Ala Ser Gly Tyr Thr Phe Thr Asp Tyr
            20              25              30
Tyr Met Asn Trp Val Arg Gln Ala Pro Gly Gln Gly Leu Glu Trp Met
```

```
                35                       40                       45
      Gly Asp Ile Ile Pro Asn His Gly Gly Ser Lys Tyr Asn Gln Lys Phe
          50                      55                      60
      Lys Asp Arg Val Thr Met Thr Thr Asp Thr Ser Thr Ser Thr Ala Tyr
          65                      70                      75                      80
      Met Glu Leu Arg Ser Leu Arg Ser Asp Asp Thr Ala Val Tyr Tyr Cys
                          85                      90                      95
      Ala Thr Trp Gly Tyr Gly Ser Ser Tyr Arg Trp Phe Asp Tyr Trp Gly
                  100                     105                     110
      Gln Gly Thr Leu Val Thr Val Ser Ser
                  115                     120
```

```
<210>   62
<211>   17
<212>   PRT
<213>   Artificial Sequence

<220>
<221>   DOMAIN
<222>   (1)..(17)
<223>   h1701-009NLG HCDR2
```

```
<400>   62
Asp Ile Ile Pro Asn Leu Gly Gly Ser Lys Tyr Asn Gln Lys Phe Lys
1                   5                   10                  15
Asp
```

```
<210>   63
<211>   17
<212>   PRT
<213>   Artificial Sequence

<220>
<221>   DOMAIN
<222>   (1)..(17)
<223>   h1701-009NVG HCDR2
```

```
<400>   63
Asp Ile Ile Pro Asn Val Gly Gly Ser Lys Tyr Asn Gln Lys Phe Lys
1                   5                   10                  15
Asp
```

```
<210>   64
<211>   17
<212>   PRT
<213>   Artificial Sequence

<220>
<221>   DOMAIN
<222>   (1)..(17)
<223>   h1701-009NNA HCDR2
```

```
<400>   64
Asp Ile Ile Pro Asn Asn Ala Gly Ser Lys Tyr Asn Gln Lys Phe Lys
1                   5                   10                  15
Asp
```

54

<210> 65
<211> 17
<212> PRT
<213> Artificial Sequence

<220>
<221> DOMAIN
<222> (1)..(17)
<223> h1701-009NMA HCDR2


<400> 65
Asp Ile Ile Pro Asn Met Ala Gly Ser Lys Tyr Asn Gln Lys Phe Lys
1               5                   10                  15
Asp


<210> 66
<211> 17
<212> PRT
<213> Artificial Sequence

<220>
<221> DOMAIN
<222> (1)..(17)
<223> h1701-009NEA HCDR2


<400> 66
Asp Ile Ile Pro Asn Glu Ala Gly Ser Lys Tyr Asn Gln Lys Phe Lys
1               5                   10                  15
Asp


<210> 67
<211> 17
<212> PRT
<213> Artificial Sequence

<220>
<221> DOMAIN
<222> (1)..(17)
<223> h1701-009NHA HCDR2


<400> 67
Asp Ile Ile Pro Asn His Ala Gly Ser Lys Tyr Asn Gln Lys Phe Lys
1               5                   10                  15
Asp


<210> 68
<211> 17
<212> PRT
<213> Artificial Sequence

<220>
<221> DOMAIN
<222> (1)..(17)
<223> h1701-009NMG HCDR2


<400> 68

```
Asp Ile Ile Pro Asn Met Gly Gly Ser Lys Tyr Asn Gln Lys Phe Lys
1               5                   10                  15
Asp
```

<210> 69
<211> 17
<212> PRT
<213> Artificial Sequence

<220>
<221> DOMAIN
<222> (1)..(17)
<223> h1701-009NEG HCDR2

<400> 69
```
Asp Ile Ile Pro Asn Glu Gly Gly Ser Lys Tyr Asn Gln Lys Phe Lys
1               5                   10                  15
Asp
```

<210> 70
<211> 17
<212> PRT
<213> Artificial Sequence

<220>
<221> DOMAIN
<222> (1)..(17)
<223> h1701-009NKG HCDR2

<400> 70
```
Asp Ile Ile Pro Asn Lys Gly Gly Ser Lys Tyr Asn Gln Lys Phe Lys
1               5                   10                  15
Asp
```

<210> 71
<211> 17
<212> PRT
<213> Artificial Sequence

<220>
<221> DOMAIN
<222> (1)..(17)
<223> h1701-009NAG HCDR2

<400> 71
```
Asp Ile Ile Pro Asn Ala Gly Gly Ser Lys Tyr Asn Gln Lys Phe Lys
1               5                   10                  15
Asp
```

<210> 72
<211> 17
<212> PRT
<213> Artificial Sequence

<220>
<221> DOMAIN

56

<222> (1)..(17)
<223> h1701-009NHG HCDR2

<400> 72
Asp Ile Ile Pro Asn His Gly Gly Ser Lys Tyr Asn Gln Lys Phe Lys
1               5                   10                  15
Asp

<210> 73
<211> 5
<212> PRT
<213> Mus musculus

<220>
<221> DOMAIN
<222> (1)..(5)
<223> anti PD-1 antibody HCDR1

<400> 73
Ser Tyr Met Met Ser
1               5

<210> 74
<211> 17
<212> PRT
<213> Mus musculus

<220>
<221> DOMAIN
<222> (1)..(17)
<223> anti PD-1 antibody HCDR2

<400> 74
Thr Ile Ser Gly Gly Gly Ala Asn Thr Tyr Tyr Pro Asp Ser Val Lys
1               5                   10                  15
Gly

<210> 75
<211> 7
<212> PRT
<213> Mus musculus

<220>
<221> DOMAIN
<222> (1)..(7)
<223> anti PD-1 antibody HCDR3

<400> 75
Gln Leu Tyr Tyr Phe Asp Tyr
1               5

<210> 76
<211> 11
<212> PRT
<213> Mus musculus

<220>

```
<221>   DOMAIN
<222>   (1)..(11)
<223>   anti PD-1 antibody LCDR1


<400>   76
Leu Ala Ser Gln Thr Ile Gly Thr Trp Leu Thr
1               5                   10


<210>   77
<211>   7
<212>   PRT
<213>   Mus musculus


<220>
<221>   DOMAIN
<222>   (1)..(7)
<223>   anti PD-1 antibody LCDR2


<400>   77
Thr Ala Thr Ser Leu Ala Asp
1               5


<210>   78
<211>   9
<212>   PRT
<213>   Mus musculus


<220>
<221>   DOMAIN
<222>   (1)..(9)
<223>   anti PD-1 antibody LCDR3


<400>   78
Gln Gln Val Tyr Ser Ile Pro Trp Thr
1               5


<210>   79
<211>   443
<212>   PRT
<213>   Artificial Sequence


<220>
<221>   CHAIN
<222>   (1)..(443)
<223>   Humanized anti PD-1 antibody  heavy chain


<400>   79
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Met Met Ser Trp Val Arg Gln Ala Pro Gly Lys Gly Leu Glu Trp Val
            35                  40                  45
Ala Thr Ile Ser Gly Gly Gly Ala Asn Thr Tyr Tyr Pro Asp Ser Val
        50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
```

```
Ala Arg Gln Leu Tyr Tyr Phe Asp Tyr Trp Gly Gln Gly Thr Thr Val
            100                     105                 110
Thr Val Ser Ser Ala Ser Thr Lys Gly Pro Ser Val Phe Pro Leu Ala
            115                 120                 125
Pro Cys Ser Arg Ser Thr Ser Glu Ser Thr Ala Ala Leu Gly Cys Leu
        130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Lys Thr Tyr Thr Cys Asn Val Asp His Lys Pro Ser Asn Thr
            195                 200                 205
Lys Val Asp Lys Arg Val Glu Ser Lys Tyr Gly Pro Pro Cys Pro Pro
        210                 215                 220
Cys Pro Ala Pro Glu Phe Leu Gly Gly Pro Ser Val Phe Leu Phe Pro
225                 230                 235                 240
Pro Lys Pro Lys Asp Thr Leu Met Ile Ser Arg Thr Pro Glu Val Thr
                245                 250                 255
Cys Val Val Val Asp Val Ser Gln Glu Asp Pro Glu Val Gln Phe Asn
            260                 265                 270
Trp Tyr Val Asp Gly Val Glu Val His Asn Ala Lys Thr Lys Pro Arg
        275                 280                 285
Glu Glu Gln Phe Asn Ser Thr Tyr Arg Val Val Ser Val Leu Thr Val
290                 295                 300
Leu His Gln Asp Trp Leu Asn Gly Lys Glu Tyr Lys Cys Lys Val Ser
305                 310                 315                 320
Asn Lys Gly Leu Pro Ser Ser Ile Glu Lys Thr Ile Ser Lys Ala Lys
                325                 330                 335
Gly Gln Pro Arg Glu Pro Gln Val Tyr Thr Leu Pro Pro Ser Gln Glu
            340                 345                 350
Glu Met Thr Lys Asn Gln Val Ser Leu Thr Cys Leu Val Lys Gly Phe
            355                 360                 365
Tyr Pro Ser Asp Ile Ala Val Glu Trp Glu Ser Asn Gly Gln Pro Glu
        370                 375                 380
Asn Asn Tyr Lys Thr Thr Pro Pro Val Leu Asp Ser Asp Gly Ser Phe
385                 390                 395                 400
Phe Leu Tyr Ser Arg Leu Thr Val Asp Lys Ser Arg Trp Gln Glu Gly
                405                 410                 415
Asn Val Phe Ser Cys Ser Val Met His Glu Ala Leu His Asn His Tyr
            420                 425                 430
Thr Gln Lys Ser Leu Ser Leu Ser Leu Gly Lys
            435                 440
```

```
<210> 80
<211> 214
<212> PRT
<213> Artificial Sequence

<220>
<221> CHAIN
<222> (1)..(214)
<223> Humanized anti PD-1 antibody light chain


<400> 80
Asp Ile Gln Met Thr Gln Ser Pro Ser Ser Leu Ser Ala Ser Val Gly
1                   5                   10                  15
Asp Arg Val Thr Ile Thr Cys Leu Ala Ser Gln Thr Ile Gly Thr Trp
            20                  25                  30
Leu Thr Trp Tyr Gln Gln Lys Pro Gly Lys Ala Pro Lys Leu Leu Ile
        35                  40                  45
```

```
Tyr Thr Ala Thr Ser Leu Ala Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Val Tyr Ser Ile Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
        130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
            165                 170                 175
Ser Thr Leu Thr Leu Ser Lys Ala Asp Tyr Glu Lys His Lys Val Tyr
        180                 185                 190
Ala Cys Glu Val Thr His Gln Gly Leu Ser Ser Pro Val Thr Lys Ser
        195                 200                 205
Phe Asn Arg Gly Glu Cys
        210


<210>  81
<211>  116
<212>  PRT
<213>  Artificial Sequence

<220>
<221>  DOMAIN
<222>  (1)..(116)
<223>  Humanized anti PD-1 antibody heavy chain variable region


<400>  81
Glu Val Gln Leu Val Glu Ser Gly Gly Gly Leu Val Gln Pro Gly Gly
1                   5                   10                  15
Ser Leu Arg Leu Ser Cys Ala Ala Ser Gly Phe Thr Phe Ser Ser Tyr
        20                  25                  30
Met Met Ser Trp Val Arg Gln Ala Pro Gly Lys Gly Leu Glu Trp Val
        35                  40                  45
Ala Thr Ile Ser Gly Gly Gly Ala Asn Thr Tyr Tyr Pro Asp Ser Val
        50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Gln Leu Tyr Tyr Phe Asp Tyr Trp Gly Gln Gly Thr Thr Val
            100                 105                 110
Thr Val Ser Ser
        115


<210>  82
<211>  107
<212>  PRT
<213>  Artificial Sequence

<220>
<221>  DOMAIN
<222>  (1)..(107)
<223>  Humanized anti PD-1 antibody light chain variable region
```

```
<400>    82
Asp Ile Gln Met Thr Gln Ser Pro Ser Ser Leu Ser Ala Ser Val Gly
1               5               10              15
Asp Arg Val Thr Ile Thr Cys Leu Ala Ser Gln Thr Ile Gly Thr Trp
            20              25              30
Leu Thr Trp Tyr Gln Gln Lys Pro Gly Lys Ala Pro Lys Leu Leu Ile
        35              40              45
Tyr Thr Ala Thr Ser Leu Ala Asp Gly Val Pro Ser Arg Phe Ser Gly
    50              55              60
Ser Gly Ser Gly Thr Asp Phe Thr Leu Thr Ile Ser Ser Leu Gln Pro
65              70              75              80
Glu Asp Phe Ala Thr Tyr Tyr Cys Gln Gln Val Tyr Ser Ile Pro Trp
            85              90              95
Thr Phe Gly Gly Gly Thr Lys Val Glu Ile Lys
        100             105
```

**Description**

**FIELD OF THE INVENTION**

The present disclosure relates to the use of a TIM-3 antibody in the preparation of medicament for treating tumor.

**BACKGROUND OF THE INVENTION**

T cell immunoglobulin mucin-domain-containing molecule 3 (TIM-3), also referred to as hepatitis A virus cellular receptor 2 (HAVCR-2), is Type I membrane surface protein, a member of TIM family. Human TIM-3 molecule is composed of 301 amino acids, comprising signal peptide, Ig variable region (IgV region), Ser/Thr-rich mucin region, trans-membrane region and cytoplasmic region; human TIM-3 shares 63% homology with murine TIM-3.

TIM-3 can regulate the function of the immune system in many ways. It can bind to ligand Gal-9 on the surface of Th1 cells to down-regulate Th1 cell response and induce Th1 cell apoptosis. It plays an important role in auto- and allogeneic immune diseases (such as systemic erythema lupus, asthma) and immune tolerance.

In addition, TIM-3 is not only expressed in immune cells, but also over-expressed in tumor cells such as ovarian cancer, meningioma, and melanoma, and directly promotes tumor growth. Down-regulating the expression of TIM-3 can significantly inhibit the invasion and metastasis of HeLa cells. The overexpression of TIM-3 is closely related to the poor prognosis of lung cancer, gastric cancer, prostate cancer and cervical cancer. In hematological tumors, TIM-3 is over-expressed on leukemia stem cells of acute myeloid leukemia and hematopoietic stem cells of MDS patients, and TIM-3+ hematopoietic stem cells have malignant biological characteristics such as low differentiation, low apoptosis and high proliferation. Therefore, inhibiting the activity of TIM-3 (such as TIM-3 antibody) to improve the function of the innate immune system is expected to become a new method for the treatment of tumors (see, for example, Ngiow et a1., Cancer Res., 71(21): 1-5 (2011); Guo et a1., Journal of Translational Medicine, 11: 215 (2013); and Ngiow et a1., Cancer Res., 71(21): 6567-6571 (2011)).

At present, TIM-3 antibodies have been reported in several patent applications, such as WO2011159877, WO2013006490, WO2015117002, WO2016144803, WO2016161270, US20150218274.

WO2018153366 (application date February 26, 2018) describes a new TIM-3 antibody with high activity, excellent affinity and stability.

As a representative of tumor immunotherapy, the effect of PD-1 antibody is obvious. Clinical data has proved that PD-1 antibody can increase the 5-year survival rate from 17% to 34% for patients with malignant tumors, and from 4% to 16% for patients with non-small cell lung cancer. However, not all patients can benefit from PD-1 antibody, or PD-1 antibody does not work at all or only maintains a short-term effect.

It has been reported in Nature Communication (February 2016) that one of the reasons for the resistance to PD-1 antibodies is that tumors have developed a new immune escape pathway, TIM-3. In the study, EGFR (T790M/L858) and KRAS (G12D) mutant lung cancer mouse models were used to construct anti-PD-1 resistant mouse models respectively. The researchers firstly analyzed the change of number of T cells in mouse tumors before treatment and after resistance to anti-PD-1 treatment; and then specifically analyzed the relationship between TIM-3 positive expression and the resistance to anti-PD-1 treatment; and found that the TIM-3 positive expression is significantly time-dependent on the duration of anti-PD-1 treatment, the positive expression of TIM-3 is low before treatment and during the treatment-sensitive period, while the positive expression of TIM-3 increases significantly after the development of drug-resistance. TIM-3 positive expression is also significantly related to the binding degree of PD-1 antibody in T cells. The higher the degree of T cell binding to PD-1 antibody, the stronger the positive expression of TIM-3 is. In conclusion, the failure of anti-PD-1 therapy is related to the up-regulation of TIM-3 expression. This molecule promotes immune escape in a way similar to PD-1/L1 by inhibiting T cell function and promoting T cell failure (Nature volume 545, pages 60-65). In addition, two TIM-3 antibodies combined with PD-1 for the treatment of malignant tumors or advanced solid tumors are in the clinical research stage (NCT02608268 and NCT02817637). For this reason, the development of new TIM-3 antibody administered alone or in combination with PD-1 for the treatment of tumors has attracted sufficient interest from pharmaceutical researchers.

**SUMMARY OF THE INVENTION**

The present disclosure provides use of a TIM-3 antibody in the preparation of a medicament for the treatment of tumors.

In some embodiments, the TIM-3 antibody or antigen-binding fragment thereof comprises one or more CDR region sequence(s) selected from the group consisting of: sequences of antibody heavy chain variable region HCDR: as shown in amino acid sequence SEQ ID NOs: 14, 15 and 16, or amino acid sequences having at least 95% sequence identity thereto; and sequences of antibody light chain variable region LCDR: as shown in amino acid sequence SEQ ID NOs: 17, 18 and 19, or amino acid sequences having at least 95% sequence identity thereto.

In some embodiments, the CDR sequences in the light and heavy chain of the TIM-3 antibody are shown in the following table:

| HCDR1 | DYYMA SEQ ID NO: 14 | LCDR1 | RASDNIYSYLA SEQ ID NO: 17 |
|-------|---------------------|-------|---------------------------|
| HCDR2 | NINYDGSSTYYLDSLKS SEQ ID NO: 15 | LCDR2 | NAKTLAE SEQ ID NO: 18 |
| HCDR3 | DVGYYGGNYGFAY SEQ ID NO: 16 | LCDR3 | QQHYGSPLT SEQ ID NO: 19 |

In some embodiments, the TIM-3 antibody or antigen-binding fragment thereof comprises one or more CDR region sequence(s) selected from the group consisting of: sequences of antibody heavy chain variable region HCDR: as shown in amino acid sequence SEQ ID NOs: 8, 43 and 10, or amino acid sequences having at least 95% sequence identity thereto; and sequences of antibody light chain variable region LCDR: as shown in amino acid sequence SEQ ID NOs: 11, 12 and 13, or amino acid sequences having at least 95% sequence identity thereto, wherein the SEQ ID NO: 43 is shown in the sequence of DIIPX$_1$X$_2$X$_3$GSKYNQKFKD: wherein, X$_1$ is selected from N, L, V, M or E, X$_2$ is selected from N, E, M, H, K, L, A or V, and X$_3$ is selected from G or A.

In other embodiments, the CDR sequences in the light and heavy chain of the TIM-3 antibody are shown in the following table:

| | Heavy chain | | Light chain |
|-------|-------------|-------|-------------|
| HCDR1 | DYYMN SEQ ID NO: 8 | LCDR1 | LASQPIGIWLA SEQ ID NO: 11 |
| HCDR2 | DIIPNNGGSKYNQKFKD SEQ ID NO: 9 | LCDR2 | AATSLAD SEQ ID NO: 12 |
| HCDR3 | WGYGSSYRWFDY SEQ ID NO: 10 | LCDR3 | QQLYSSPWT SEQ ID NO: 13 |

Preferably, in some embodiments, the TIM-3 antibody or antigen-binding fragment thereof is selected from the group consisting of murine antibody, chimeric antibody, humanized antibody or antigen-binding fragment thereof.

In some embodiments, the light chain and heavy chain FR region sequence(s) of the humanized antibody light chain and heavy chain variable region(s) is/are respectively derived from human germline light chain and heavy chain or the mutant sequence(s) thereof.

Further, in some embodiments, the humanized antibody comprises heavy chain variable region as shown in SEQ ID NO: 31 or variant thereof, and preferably the variant comprises 1 to 10 amino acid alternation(s) when compared with heavy chain variable region as shown in SEQ ID NO: 31, more preferably the amino acid alternations are amino acid back-mutations Q3K and R87K; and the humanized antibody comprises light chain variable region as shown in SEQ ID NO: 32 or variant thereof, and preferably the variant comprises 1 to 10 amino acid alternation(s) when compared with light chain variable region as shown in SEQ ID NO: 32, more preferably the amino acid alternation is selected from the group consisting of amino acid back-mutations Q3K and I48V, K45Q, A43S and T85S.

The sequences of the humanized antibody heavy and light chain variable region described above are as follows:

Sequence of heavy chain variable region, SEQ ID NO: 31

*EVQLVESGGGLVQPGGSLRLSCAASGFTFS*DYYMA*WVRQAPGKGLEWVA*NINYDG SSTYYLDSLKS*RFTISRDNAKNSLYLQMNSLRAEDTAVYYCAR*DVGYYGGNYGFAY *WGQGTLVTVSS;*

Sequence of light chain variable region, SEQ ID NO: 32

*DIQMTQSPSSLSASVGDRVTITC*RASDNIYSYLA*WYQQKPGKAPKLLIY*NAKTLAE*G VPSRFSGSGSGTDFTLTISSLQPEDFATYYC*QQHYGSPLT*FGQGTKLEIK;*

Note: The arrangement is FR1-CDR1-FR2-CDR2-FR3-CDR3-FR4, the italics in the sequence represent the FR sequences, and the underline represents the CDR sequences.

In some embodiments, the sequences of the humanized TIM-3 antibody heavy and light chain variable regions are as follows:

heavy chain variable region sequence SEQ ID NO: 33:

EVQLVESGGGLVQPGGSLRLSCAASGFTFSDYYMAWVRQAPGKGLEWVANINY
DGSSTYYLDSLKSRFTISRDNAKNSLYLQMNSLRAEDTAVYYCARDVGYYGGN
YGFAYWGQGTLVTVSS;

light chain variable region sequence SEQ ID NO: 36:

DIQMTQSPSSLSASVGDRVTITCRASDNIYSYLAWYQQKPGKAPKLLIYNAKTL
AEGVPSRFSGSGSGTDFTLTISSLQPEDFATYYCQQHYGSPLTFGQGTKLEIK.

In some embodiments, the humanized antibody comprises heavy chain variable region as shown in SEQ ID NO: 20 or variant thereof, and preferably the variant comprises 1 to 10 amino acid alternation(s) when compared with heavy chain variable region as shown in SEQ ID NO: 20, more preferably the amino acid alternations are amino acid back-mutations D89E, R98T, G49A, M48I, M70L, R38K and V68A; and the humanized antibody comprises light chain variable region as shown in SEQ ID NO: 21 or variant thereof, and preferably the variant comprises 1 to 10 amino acid alternation(s) when compared with light chain variable region as shown in SEQ ID NO: 21, more preferably the amino acid alternation is amino acid back-mutation A43S.

The sequences of the humanized antibody heavy and light chain variable regions described above are as follows:

heavy chain variable region sequence SEQ ID NO: 20

*QVQLVQSGAEVKKPGASVKVSCKASGYTFT*DYYMN*WVRQAPGQGLEWMG*DIIPNN
GGSKYNQKFKD*RVTMTTDTSTSTAYMELRSLRSDDTAVYYCAR*WGYGSSYRWFDY*WG
QGTLVTVSS;*

light chain variable region sequence SEQ ID NO: 21

*DIQMTQSPSSLSASVGDRVTITC*LASQPIGIWLA*WYQQKPGKAPKLLIY*AATSLAD*GVP
SRFSGSGSGTDFTFTISSLQPEDIATYYC*QQLYSSPWT*FGGGTKVEIK;*

Note: The arrangement is FR1-CDR1-FR2-CDR2-FR3-CDR3-FR4, the italics in the sequence represent the FR sequences, and the underline represents the CDR sequences.

In some embodiments, the sequences of the humanized TIM-3 antibody heavy and light chain variable regions are as follows:

heavy chain variable region sequence SEQ ID NO: 51

QVQLVQSGAEVKKPGASVKVSCKASGYTFTDYYMNWVRQAPGQGLEWMGDII
PNLGGSKYNQKFKDRVTMTTDTSTSTAYMELRSLRSDDTAVYYCATWGYGSSY
RWFDYWGQGTLVTVSS;

light chain variable region sequence SEQ ID NO: 29

DIQMTQSPSSLSASVGDRVTITCLASQPIGIWLAWYQQKPGKAPKLLIYAATSLA
DGVPSRFSGSGSGTDFTFTISSLQPEDIATYYCQQLYSSPWTFGGGTKVEIK.

Preferably, the TIM-3 antibody is a full-length antibody, further comprising human antibody constant region(s), preferably comprising human heavy chain constant region sequence as shown in SEQ ID NO: 41 and preferably human light chain constant region as shown in SEQ ID NO: 42.

The heavy chain constant region sequence is as shown in SEQ ID NO: 41:

ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTKSLSLSLG
K;

The light chain constant region sequence is as shown in SEQ ID NO: 42:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC.

In some embodiments, the antigen-binding fragment of the TIM-3 antibody is selected from the group consisting of Fab, Fab', F(ab')2, single-chain antibody (scFv), dimerized V region (diabody), disulfide bond stabilized V region (dsFv), and antigen-binding fragment of peptide containing CDRs.

In another aspect, the TIM-3 antibody or antigen-binding fragment thereof described in the use according to present disclosure is administered in combination with an anti-PD-1 antibody or antigen-binding fragment thereof.

Anti-PD-1 antibody is known and can be selected from but not limited to: AMP-224, GLS-010, IBI-308, REGN-2810, PDR-001, BGB-A317, Pidilizumab, PF-06801591, Genolimzumab, CA-170, MEDI-0680, JS-001, TSR-042, Camrelizumab, Pembrolizumab, LZM-009, AK-103 and Nivolumab.

Preferably, the light chain variable region of the PD-1 antibody comprises LCDR1, LCDR2 and LCDR3 as shown in SEQ ID NO: 76, SEQ ID NO: 77 and SEQ ID NO: 78, respectively; the heavy chain variable region of the anti-PD-1 antibody comprises HCDR1, HCDR2 and HCDR3 as shown in SEQ ID NO: 73, SEQ ID NO: 74 and SEQ ID NO: 75, respectively.

In other embodiments, each CDR sequence of the anti-PD-1 antibody is shown in the following table:

| Name | Sequence | SEQ ID NO |
|------|----------|-----------|
| HCDR1 | SYMMS | SEQ ID NO: 73 |
| HCDR2 | TISGGGANTYYPDSVKG | SEQ ID NO: 74 |
| HCDR3 | QLYYFDY | SEQ ID NO: 75 |
| LCDR1 | LASQTIGTWLT | SEQ ID NO: 76 |
| LCDR2 | TATSLAD | SEQ ID NO: 77 |
| LCDR3 | QQVYSIPWT | SEQ ID NO: 78 |

Preferably, the anti-PD-1 antibody is a humanized antibody or fragment thereof.

In an alternative embodiment, the antigen-binding fragment of the anti-PD-1 antibody in the present disclosure is antibody fragment selected from the group consisting of Fab, Fab'-SH, Fv, scFv, and (Fab') 2 fragment.

The immunoglobulin can be derived from any commonly known isotype, including but not limited to IgA, secreted IgA, IgG, and IgM. The subclasses of IgG are also well known to those skilled in the art, including but not limited to IgG1, IgG2, IgG3, and IgG4. "Isotype" refers to Ab class or subclass (for example, IgM or IgG1) encoded by the heavy chain constant region gene. In some alternative embodiments, the anti-PD-1 antibody or antigen-binding fragment thereof in the present disclosure comprises heavy chain constant region(s) of human IgG1, IgG2, IgG3, or IgG4 isotype, and preferably comprises heavy chain constant region(s) of IgG1 or IgG4 isotype.

In other alternative embodiments, the anti-PD-1 antibody or antigen-binding fragment thereof comprises light chain constant region(s) of kappa or lambda.

Further, preferably the sequence of the humanized antibody light chain variable region is the sequence as shown in SEQ ID NO: 82 or variant thereof, and preferably the variant has 0-10 amino acid alternation(s) in the light chain variable region, more preferably the amino acid alternation is A43S; and the sequence of the humanized antibody heavy chain variable region is as shown in SEQ ID NO: 81 or variant thereof, and preferably the variant has 0-10 amino acid alternation(s) in the heavy chain variable region, more preferably the amino acid alternation is G44R.

In some embodiments, the sequences of the humanized anti-PD-1 antibody heavy and light chain variable regions are as follows:

Heavy chain variable region

EVQLVESGGGLVQPGGSLRLSCAASGFTFSSYMMSWVRQAPGKGLEWVATISG
GGANTYYPDSVKGRFTISRDNAKNSLYLQMNSLRAEDTAVYYCARQLYYFDYW
GQGTTVTVSS

SEQ ID NO: 81;

Light chain variable region

DIQMTQSPSSLSASVGDRVTITCLASQTIGTWLTWYQQKPGKAPKLLIYTATSLA
DGVPSRFSGSGSGTDFTLTISSLQPEDFATYYCQQVYSIPWTFGGGTKVEIK

SEQ ID NO: 82.

Preferably, the humanized anti-PD-1 antibody light chain sequence is the sequence as shown in SEQ ID NO: 80 or variant thereof; preferably the variant has 0-10 amino acid alternation(s) in the light chain variable region, more preferably the amino acid alternation is A43S; the humanized antibody heavy chain sequence is the sequence as shown in SEQ ID NO: 79 or variant thereof, preferably the variant has 0-10 amino acid alternation(s) in the heavy chain variable region, more preferably the amino acid alternation is G44R.

In another embodiment, the light chain sequence of the humanized anti-PD-1 antibody is the sequence as shown in SEQ ID NO: 80, and the heavy chain sequence is the sequence as shown in SEQ ID NO: 79:

Heavy chain

EVQLVESGGGLVQPGGSLRLSCAASGFTFSSYMMSWVRQAPGKGLEWVATISG
GGANTYYPDSVKGRFTISRDNAKNSLYLQMNSLRAEDTAVYYCARQLYYFDYW
GQGTTVTVSSASTKGPSVFPLAPCSRSTSESTAALGCLVKDYFPEPVTVSWNSGA
LTSGVHTFPAVLQSSGLYSLSSVVTVPSSSLGTKTYTCNVDHKPSNTKVDKRVES
KYGPPCPPCPAPEFLGGPSVFLFPPKPKDTLMISRTPEVTCVVVDVSQEDPEVQF
NWYVDGVEVHNAKTKPREEQFNSTYRVVSVLTVLHQDWLNGKEYKCKVSNK
GLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNH
YTQKSLSLSLGK

SEQ ID NO: 79;

Light chain

DIQMTQSPSSLSASVGDRVTITCLASQTIGTWLTWYQQKPGKAPKLLIYTATSLA
DGVPSRFSGSGSGTDFTLTISSLQPEDFATYYCQQVYSIPWTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 80.

The anti-PD-1 antibody combined with the TIM-3 antibody described in the present disclosure exhibits pharmaceutical synergistic effect in the preparation of medicament for the treatment of tumors.

Depending on the type and severity of the disease, the administration dosage in human subjects of the TIM-3 antibody or antigen-binding fragment thereof described herein (administered according to the weight of the patient) is 0.1 to 10.0 mg/kg, which can be 0.1 mg/kg, 0.2mg/kg, 0.3mg/kg, 0.4mg/kg, 0.5mg/kg, 0.6mg/kg, 0.7mg/kg, 0.8mg/kg, 0.9mg/kg, 1.0mg/kg, 1.2 mg/kg, 1.4mg/kg, 1.6mg/kg, 1.8mg/kg, 2.0mg/kg, 2.2mg/kg, 2.4mg/kg, 2.6mg/kg, 2.8mg/kg, 3.0mg/kg, 3.2 mg/kg, 3.4mg/kg, 3.6mg/kg, 3.8mg/kg, 4.0mg/kg, 4.2mg/kg, 4.4mg/kg, 4.6mg/kg, 4.8mg/kg, 5.0mg/kg, 5.2 mg/kg, 5.4mg/kg, 5.6mg/kg, 5.8mg/kg, 6.0mg/kg, 6.2mg/kg, 6.4mg/kg, 6.6mg/kg, 6.8mg/kg, 7.0mg/kg, 7.2mg/kg, 7.4mg/kg, 7.6mg/kg, 7.8mg/kg, 8.0mg/kg, 8.2mg/kg, 8.4mg/kg, 8.6mg/kg, 8.8mg/kg, 9.0mg/kg, 9.2 mg/kg, 9.4mg/kg, 9.6mg/kg, 9.8mg/kg, 10.0mg/kg.

In an alternative embodiment, the administration dosage in human subjects of the TIM-3 antibody or antigen-binding fragment thereof (administered according to the weight of the patient) is 1 mg to 1000 mg, which can be 1.0mg, 1.2mg, 1.4mg, 1.6mg, 1.8mg, 2.0mg, 2.2mg, 2.4mg, 2.6mg, 2.8mg, 3.0mg, 3.2mg, 3.4mg, 3.6mg, 3.8mg, 4.0mg, 4.2mg, 4.4mg, 4.6mg, 4.8mg, 5.0mg, 5.2mg, 5.4mg, 5.6mg, 5.8mg, 6.0mg, 6.2mg, 6.4mg, 6.6mg, 6.8mg, 7.0mg, 7.2mg, 7.4mg, 7.6mg, 7.8mg, 8.0mg, 8.2mg, 8.4mg, 8.6mg, 8.8mg, 9.0mg, 9.2mg, 9.4mg, 9.6mg, 9.8mg, 10.0mg, 15mg, 20mg, 25mg, 30mg, 35mg, 40mg, 45mg, 50mg, 55mg, 60mg, 65mg, 70mg, 75mg, 80mg, 85mg, 90mg, 95mg, 100mg, 105mg, 110mg, 115mg, 120mg, 125mg, 130mg, 135mg, 140mg, 145mg, 150mg, 155mg, 160mg, 165mg, 170mg, 175mg, 180mg, 185mg, 190mg, 195mg, 200mg, 205mg, 210mg, 215mg, 220mg, 225mg, 230mg, 235mg, 240mg, 245mg, 250mg, 255mg, 260mg, 265mg, 270mg, 275mg, 280mg, 285mg, 290mg, 295mg, 300mg, 305mg, 310mg, 315mg, 320mg, 325mg, 330mg, 335mg, 340mg, 345mg, 350mg, 355mg, 360mg, 365mg, 370mg, 375mg, 380mg, 385mg, 390mg, 395mg, 400mg, 405mg, 410mg, 415mg, 420mg, 425mg, 430mg, 435mg, 440mg, 445mg, 450mg, 455mg, 460mg, 465mg, 470mg, 475mg, 480mg, 485mg, 490mg, 495mg, 500mg, 505mg, 510mg, 515mg, 520mg, 525mg, 530mg, 535mg, 540mg, 545mg, 550mg, 555mg, 560mg, 565mg, 570mg, 575mg, 580mg, 585mg, 590mg, 595mg, 600mg, 605mg, 610mg, 615mg, 620mg, 625mg, 630mg, 635mg, 640mg, 645mg, 650mg, 655mg, 660mg, 665mg, 670mg, 675mg, 680mg, 685mg, 690mg, 695mg, 700mg, 705mg, 710mg, 715mg, 720mg, 725mg, 730mg, 735mg, 740mg, 745mg, 750mg, 755mg, 760mg, 765mg, 770mg, 775mg, 780mg, 785mg, 790mg, 795mg, 800mg, 805mg, 810mg, 815mg, 820mg, 825mg, 830mg, 835mg, 840mg, 845mg, 850mg, 855mg, 860mg, 865mg, 870mg, 875mg, 880mg, 885mg, 890mg, 895mg, 900mg, 905mg, 910mg, 915mg, 920mg, 925mg, 930mg, 935mg, 940mg, 945mg, 950mg, 955mg, 960mg, 965mg, 970mg, 975mg, 980mg, 985mg, 990mg, 995mg, 1000mg, preferably 50 to 600mg, most preferably 200mg.

The administration frequency will vary with the type and severity of the disease. In some embodiments, the administration frequency of the TIM-3 antibody or antigen-binding fragment thereof described in the present disclosure is once a week, once every two weeks, once every three weeks, once every four weeks, once every six weeks, or once every eight weeks.

In an alternative embodiment, the TIM-3 antibody or antigen-binding fragment thereof described in the present disclosure is administered in a human subject at a dosage of 50 to 600 mg/once every 2-3 weeks. However, other dosage may be useful, preferably 200 mg/once every 2-3 weeks.

The administration dosage in a human subject of the anti-PD-1 antibody or antigen-binding fragment thereof described herein is 0.1 to 10.0 mg/kg, which can be 0.1mg/kg, 0.2mg/kg, 0.3mg/kg , 0.4mg/kg, 0.5mg/kg, 0.6mg/kg, 0.7mg/kg, 0.8mg/kg, 0.9mg/kg, 1.0mg/kg, 1.2mg/kg, 1.4mg/kg, 1.6mg/kg , 1.8mg/kg, 2.0mg/kg, 2.2mg/kg, 2.4mg/kg, 2.6mg/kg, 2.8mg/kg, 3.0mg/kg, 3.2mg/kg, 3.4mg/kg, 3.6mg/kg, 3.8mg/kg, 4.0mg/kg, 4.2mg/kg, 4.4mg/kg, 4.6mg/kg, 4.8mg/kg, 5.0mg/kg, 5.2mg/kg, 5.4mg/kg, 5.6mg/kg, 5.8mg/kg, 6.0mg/kg, 6.2mg/kg, 6.4mg/kg, 6.6mg/kg, 6.8mg/kg, 7.0mg/kg, 7.2mg/kg, 7.4mg/kg, 7.6mg/kg, 7.8mg/kg, 8.0mg/kg, 8.2mg/kg, 8.4mg/kg, 8.6mg/kg, 8.8mg/kg, 9.0mg/kg, 9.2mg/kg, 9.4mg/kg, 9.6mg/kg, 9.8mg/kg, 10.0mg/kg.

In an alternative embodiment, the administration dosage in a human subject of the anti-PD-1 antibody or antigen-binding fragment thereof is 1 mg to 1000 mg, which can be 1.0mg, 1.2mg, 1.4mg, 1.6mg, 1.8mg, 2.0mg, 2.2mg, 2.4mg, 2.6mg, 2.8mg, 3.0mg, 3.2mg, 3.4mg, 3.6mg, 3.8mg, 4.0mg, 4.2mg, 4.4mg, 4.6mg, 4.8mg, 5.0mg, 5.2mg, 5.4mg, 5.6mg, 5.8mg, 6.0mg, 6.2mg, 6.4mg, 6.6mg, 6.8mg, 7.0mg, 7.2mg, 7.4mg, 7.6mg, 7.8mg, 8.0mg, 8.2mg, 8.4mg, 8.6mg, 8.8mg, 9.0mg, 9.2mg, 9.4mg, 9.6mg, 9.8mg, 10.0mg, 15mg, 20mg, 25mg, 30mg, 35mg, 40mg, 45mg, 50mg, 55mg, 60mg, 65mg, 70mg, 75mg, 80mg, 85mg, 90mg, 95mg, 100mg, 105mg, 110mg, 115mg, 120mg, 125mg, 130mg, 135mg, 140mg, 145mg, 150mg, 155mg, 160mg, 165mg, 170mg, 175mg, 180mg, 185mg, 190mg, 195mg, 200mg, 205mg, 210mg, 215mg, 220mg, 225mg, 230mg, 235mg, 240mg, 245mg, 250mg, 255mg, 260mg, 265mg, 270mg, 275mg, 280mg, 285mg, 290mg, 295mg, 300mg, 305mg, 310mg, 315mg, 320mg, 325mg, 330mg, 335mg, 340mg, 345mg, 350mg, 355mg, 360mg, 365mg, 370mg, 375mg, 380mg, 385mg, 390mg, 395mg, 400mg, 405mg, 410mg, 415mg, 420mg, 425mg, 430mg, 435mg, 440mg, 445mg, 450mg, 455mg, 460mg, 465mg, 470mg, 475mg, 480mg, 485mg, 490mg, 495mg, 500mg, 505mg, 510mg, 515mg, 520mg, 525mg, 530mg, 535mg, 540mg, 545mg, 550mg, 555mg, 560mg, 565mg, 570mg, 575mg, 580mg, 585mg, 590mg, 595mg, 600mg, 605mg, 610mg, 615mg, 620mg, 625mg, 630mg, 635mg, 640mg, 645mg, 650mg, 655mg, 660mg, 665mg, 670mg, 675mg, 680mg, 685mg, 690mg, 695mg, 700mg, 705mg, 710mg, 715mg, 720mg, 725mg, 730mg, 735mg, 740mg, 745mg, 750mg, 755mg, 760mg, 765mg, 770mg, 775mg, 780mg, 785mg, 790mg, 795mg, 800mg, 805mg, 810mg, 815mg, 820mg, 825mg, 830mg, 835mg, 840mg, 845mg, 850mg, 855mg, 860mg, 865mg, 870mg, 875mg, 880mg, 885mg, 890mg, 895mg, 900mg, 905mg, 910mg, 915mg, 920mg, 925mg, 930mg, 935mg, 940mg, 945mg, 950mg, 955mg, 960mg, 965mg, 970mg, 975mg, 980mg, 985mg, 990mg, 995mg, 1000mg, preferably 50 to 600mg, most preferably 200mg.

The administration frequency will vary with the type and severity of the disease. In some embodiments, the administration frequency of the anti-PD-1 antibody or antigen-binding fragment thereof described in the present disclosure is once a week, once every two weeks, once every three weeks, once every four weeks, once every six weeks, or once every eight weeks.

In an alternative embodiment, the anti-PD-1 antibody or antigen-binding fragment thereof described in the present disclosure is administered at a dosage of 50 to 600 mg/once every 2-3 weeks. However, other dosage may be useful, preferably 200 mg/once every 2-3 weeks.

In some embodiments, the administration dosage in a human subject of the TIM-3 antibody or antigen-binding fragment thereof (administered according to the weight of the patient) is 0.1 to 10.0 mg/kg, and administration dosage of the anti-PD-1 antibody or antigen-binding fragment thereof is 0.1 to 10.0mg/kg.

In some embodiments, the administration dosage in a human subject of the TIM-3 antibody or antigen-binding fragment thereof is 1 to 1000 mg, and the administration dosage of the anti-PD-1 antibody or antigen-binding fragment thereof is 1 to 1000mg, once every three weeks.

In some embodiments, the administration dosage in a human subject of the TIM-3 antibody or antigen-binding fragment thereof (administered according to the weight of the patient) is 1 to 1000 mg, and the administration dosage of the anti-PD-1 antibody or antigen-binding fragment thereof is 50 to 600 mg, once every three weeks.

In some embodiments, the administration dosage in a human subject of the TIM-3 antibody or antigen-binding fragment thereof (administered according to the weight of the patient) is 1 to 1000 mg, once every three weeks; and the administration dosage in a human subject of the anti-PD-1 antibody or antigen-binding fragment thereof (administered according to the weight of the patient) is 1 to 1000 mg.

The administration route in the present disclosure may be oral administration, parenteral administration, transdermal administration; the parenteral administration comprises but not limited to intravenous injection, subcutaneous injection, or intramuscular injection.

In a preferred embodiment of the present disclosure, the PD-1 antibody is administered by injection, such as subcutaneous or intravenous injection, and the PD-1 antibody must be formulated into an injectable form before injection. In particular, preferably the injectable form of the PD-1 antibody is injection solution or lyophilized powder, which comprises PD-1 antibody, buffer, stabilizer, and optionally surfactant. The buffer can be one or more selected from the group consisting of acetate, citrate, succinate and phosphate. The stabilizer may be selected from saccharides or amino acids, preferably disaccharides, such as sucrose, lactose, trehalose, and maltose. The surfactant is selected from the group consisting of polyoxyethylene hydrogenated castor oil, fatty acid glycerides, polyoxyethylene sorbitan fatty acid esters, preferably the polyoxyethylene sorbitan fatty acid ester is polysorbate 20, 40, 60 or 80, most preferably polysorbate 20. The most preferably injectable form of PD-1 antibody comprises PD-1 antibody, acetate buffer, trehalose and polysorbate 20.

The present disclosure also provides a pharmaceutical kit, or a pharmaceutical composition, which comprises the anti-TIM-3 antibody or antigen-binding fragment thereof and the anti-PD-1 antibody or antigen-binding fragment thereof.

The present disclosure also provides a method for treating tumors, comprising administering a therapeutically effective amount of the TIM-3 antibody or antigen-binding fragment thereof or/and the anti-PD-1 antibody or antigen-binding fragment thereof to a patient with tumor.

Examples of tumors described in the use of the present disclosure are selected from the group consisting of, but not limited to: breast cancer (such as triple negative breast cancer), lung cancer, gastric cancer, colorectal cancer (such as rectal cancer, colorectal cancer), kidney cancer (such as renal cell carcinoma), liver cancer (such as hepatocellular carcinoma), melanoma (such as metastatic melanoma), non-small cell lung cancer, lymphoblastic T-cell leukemia, chronic myeloid leukemia, chronic lymphocytic leukemia, hairy cell leukemia, acute lymphoblastic leukemia, acute myeloid leukemia (AML), chronic neutrophil leukemia, acute lymphoblastic T-cell leukemia, immunoblastic mast cell leukemia, mantle cell leukemia, multiple myeloma megakaryoblastic leukemia, acute megakaryocytic leukemia, promyelocytic leukemia, erythroleukemia, malignant lymphoma, multiple myeloma, plasmacytoma, Hodgkin's lymphoma, non-Hodgkin's lymphoma, lymphoblastic T-cell lymphoma, burkitt's lymphoma, follicular lymphoma and myelodysplastic syndrome (MDS).

In an alternative embodiment, the tumor in the use of the present disclosure is non-small cell lung cancer, breast cancer (such as triple negative breast cancer), melanoma (such as metastatic melanoma), kidney cancer, colorectal cancer or liver cancer, preferably colorectal cancer or non-small cell lung cancer.

Otherwise indicated specifically, the terms in the present disclosure have the following definition:

In the present disclosure, the so-called "in combination with" is a way of administration, which means that at least one dosage of the TIM-3 antibody or antigen-binding fragment thereof and at least one dosage of the anti-PD-1 antibody or antigen-binding fragment thereof are provided within given time period, in which both medicaments show pharmacological effect to produce pharmacological efficacy. This time period can be one dosing cycle. The two medicaments can be administered simultaneously or sequentially.

The "humanized antibody" used in the present disclosure, also known as CDR-grafted antibody, refers to an antibody generated by grafting mouse CDR sequences onto the human antibody variable region frameworks (i.e. antibodies produced within different types of human germline antibody framework sequences). Humanized antibodies overcome the strong antibody response induced by the chimeric antibody which carries a large amount of mouse protein components. Such framework sequences can be obtained from public DNA databases or published references that include germline antibody gene sequences. For example, the germline DNA sequences of human heavy chain and light chain variable region genes can be found in the "VBase" human germline sequence database (available on the Internet www.mrccpe.com.ac.uk/vbase), as well as in Kabat, EA, etc., 1991 Sequences of Proteins of Immunological Interest, 5th edition. In a preferred embodiment of the present disclosure, the CDR sequence of the PD-1 humanized antibody is selected from the group consisting of SEQ ID NO: 73, 74, 75, 76, 77 and 78.

The "murine antibody" used in the present disclosure is a monoclonal antibody against human TIM-3, which is prepared according to the knowledge and skills in the art. During the preparation, a test subject is injected with TIM-3 antigen, and then hybridoma expressing antibody which possesses desired sequences or functional characteristics is separated. In some preferred embodiments of the present invention, the murine TIM-3 antibody or antigen-binding fragment thereof further comprises light chain constant region(s) of murine κ, λ chain or variants thereof, or further comprises heavy chain constant region(s) of murine IgG1, IgG2, IgG3, or variants thereof.

The "chimeric antibody" used in the present disclosure is an antibody which is formed by fusing the variable region of a murine antibody with the constant region of a human antibody, the chimeric antibody can alleviate the murine antibody-induced immune response. To establish a chimeric antibody, hybridoma secreting specific murine monoclonal antibody is firstly established, a variable region gene is cloned from mouse hybridoma cells, then a constant region gene of a human antibody is cloned as desired, the mouse variable region gene is ligated to the human constant region gene to form a chimeric gene which can be then inserted into an expression vector, and finally the chimeric antibody molecule is expressed in an eukaryotic or prokaryotic system. In a preferred embodiment of the present invention, the antibody light chain of the TIM-3 chimeric antibody further comprises light chain constant region(s) of human κ, λ chain or variant thereof. The antibody heavy chain of the TIM-3 chimeric antibody further comprises heavy chain constant region(s) of human IgG1, IgG2, IgG3, IgG4 or variant thereof, preferably comprises the human IgG1, IgG2 or IgG4 heavy chain constant region(s), or comprises IgG1, IgG2 or IgG4 variants comprising amino acid mutation(s) (such as YTE mutation or back-mutation).

The "antigen-binding fragment" of the anti-PD-1 antibody used in the present disclosure refers to Fab fragment, Fab' fragment, F(ab')2 fragment having antigen-binding activity, as well as Fv fragment, scFv fragment binding to human PD-1; the "antigen-binding fragment" comprises one or more CDR region(s) selected from SEQ ID NO: 1 to SEQ ID NO: 6 of the antibody described in the present disclosure. Fv fragment is a minimum antibody fragment carrying all antigen-binding sites, it comprises antibody heavy chain variable region and light chain variable region, but without constant region. Generally, Fv antibody further comprises a polypeptide linker between the VH and VL domains, and is capable of forming a structure necessary for antigen binding. Also, different linkers can be used to connect the variable regions of two antibodies to form a polypeptide chain, namely single chain antibody or single chain Fv (sFv). The term "binding to PD-1" in the present disclosure refers to the ability to interact with human PD-1. The term "antigen-binding site" in the present disclosure refers to discrete three-dimensional sites on the antigen that is recognized by the antibody or antigen-binding fragment of the present disclosure.

The "antigen-binding fragment" or "functional fragment" of the TIM-3 antibody described in the present disclosure refers to one or more fragment(s) of the antibody that retain(s) the ability to specifically bind to an antigen (for example, TIM-3). It has been shown that fragments of full-length antibody can be used to perform the antigen-binding function of antibody. Examples of the binding fragment contained in the term "antigen-binding fragment" of the antibody include (i) Fab fragment, a monovalent fragment composed of VL, VH, CL and CH1 domains; (ii) F(ab')2 fragment, a bivalent fragment including two Fab fragments connected by a disulfide bridge on the hinge region, (iii) Fd fragment composed of VH and CH1 domains; (iv) Fv fragment composed of VH and VL domains from one arm of an antibody; (v) single domain or dAb fragment (Ward et al., (1989) Nature 341: 544-546), which is composed of VH domain; and (vi) isolated complementary determining region (CDR) or (vii) combination of two or more isolated CDRs, optionally connected by synthetic linkers. In addition, although the two domains VL and VH of the Fv fragment are encoded by separate genes, recombination methods can be used to connect them through a synthetic linker so that a single protein chain can be produced in which the VL and VH regions are matched with each other to form a monovalent molecule (referred to as single chain Fv (scFv); see, for example, Bird et al. (1988) Science 242: 423-426; and Huston et al. (1988) Proc. Natl. Acad. Sci USA 85: 5879-5883). Such single chain antibody are also intended to be included in the term "antigen-binding fragment" of antibody. Such antibody fragments are obtained using conventional techniques known to those skilled in the art, and the fragments are screened for their function in the same manner as that for intact antibodies. The antigen binding portion can be produced by recombinant DNA technology or by enzymatic or chemical fragmentation of the intact immunoglobulin. The antibodies may be antibodies of different isotypes, for example IgG (for example, IgG1, IgG2, IgG3 or IgG4 subtypes), IgA1, IgA2, IgD, IgE or IgM antibody.

Fab is an antibody fragment that has a molecular weight of about 50,000 and has antigen-binding activity, which is obtained by treating IgG antibody molecules with the protease papain (cleaving the amino acid residue at position 224 of the H chain), wherein about half of the H chain at its N-terminal side and the entire L chain are connected together by disulfide bond.

The Fab described in the present disclosure can be produced by treating the monoclonal antibody of the present invention (that specifically recognizes human TIM-3 and binds to the amino acid sequence of the extracellular region or its three-dimensional structure) with papain. In addition, the Fab can be produced by inserting the DNA encoding the Fab of the antibody into a prokaryotic expression vector or a eukaryotic expression vector and introducing the vector into a prokaryotic or eukaryotic organism to express the Fab.

F(ab')2 is an antibody fragment with a molecular weight of about 100,000 obtained by digesting the part downstream of the two disulfide bonds in the IgG hinge region with the pepsin enzyme, F(ab')2 has antigen binding activity and comprises two Fab regions connected at the position of hinge.

The F(ab')2 described in the present disclosure can be produced by treating the monoclonal antibody of the present invention (that specifically recognizes human TIM-3 and binds to the amino acid sequence of the extracellular region or its three-dimensional structure) with pepsin. In addition, the F(ab')2 can be produced by linking Fab' described below with a thioether bond or a disulfide bond.

Fab' is an antibody fragment with a molecular weight of about 50,000 and having antigen-binding activity, which is obtained by cleaving the disulfide bond in the hinge region of F(ab')2. The Fab' of the present invention can be produced by treating the F(ab')2 of the present invention (that specifically recognizes TIM-3 and binds to the amino acid sequence of the extracellular region or its three-dimensional structure) with a reducing agent such as dithiothreitol.

In addition, the Fab' can be produced by inserting DNA encoding the Fab' fragment of the antibody into a prokaryotic expression vector or a eukaryotic expression vector and introducing the vector into a prokaryotic organism or eukaryotic organism to express Fab'.

The "single chain antibody", "single chain Fv" or "scFv" described in the present disclosure refers to a molecular in which the antibody heavy chain variable domain (or region; VH) is connected to the antibody light chain variable domain (or region; VL) with a linker. Such scFv molecules have general structure: $NH_2$-VL-linker-VH-COOH or $NH_2$-VH-linker-VL-COOH. A suitable linker in prior art consists of repeated GGGGS amino acid sequence(s) or variant thereof, for example 1-4 repeated variants can be used (Holliger et al. (1993), Proc. Natl. Acad. Sci. USA 90: 6444-6448). Other linkers that can be used in the present invention are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol.

The scFv described in the present disclosure can be produced by the following steps: producing cDNA encoding VH and VL of the monoclonal antibody of the present invention (that specifically recognizes human TIM-3 and binds to the amino acid sequence of the extracellular region or its three-dimensional structure), constructing DNA encoding the scFv, inserting the DNA into a prokaryotic expression vector or a eukaryotic expression vector, and then introducing the expression vector into a prokaryote or eukaryotic organism to express the scFv.

The "effective amount" described in the present disclosure comprises an amount sufficient to improve or prevent the symptoms or conditions of the medical condition. An effective amount also refers to an amount sufficient to allow or facilitate diagnosis. The effective amount for a particular patient or veterinary subject can vary depending on factors such as the condition to be treated, the patient's general health, administration method, route and dosage, and the severity of side effects. The effective amount can be the maximum dosage or dosing schedule that avoids significant side effects or toxic effects.

The "CDR" described in the present disclosure refers to one of the six hypervariable regions within the variable domain of an antibody that mainly contribute to antigen binding. One of the most commonly used definition of the 6 CDRs is provided by Kabat E.A. et al. (1991) Sequences of proteins of immunological interest. NIH Publication 91-3242). As used herein, the Kabat definition of CDR only applies to the CDR1, CDR2, and CDR3 of the light chain variable domain (CDR L1, CDR L2, CDR L3 or L1, L2, L3), and the CDR2 and CDR3 of the heavy chain variable domain (CDR H2, CDR H3 or H2, H3).

The engineered antibody or antigen-binding fragment in the present disclosure can be prepared and purified by conventional methods. For example, the cDNA sequences encoding the heavy and light chains can be cloned and recombined into a GS expression vector. The recombinant immunoglobulin expression vector can be stably transfected into CHO cells. As a more recommended prior art, mammalian expression systems can lead to glycosylation of antibodies, especially at the highly conserved N-terminal sites in the Fc region. Stable clones are obtained by expressing antibodies that specifically bind to human TIM-3. Positive clones are expanded in the serum-free medium of the bioreactor to produce antibodies. The culture medium comprising secreted antibody can be purified by conventional techniques. For example, A- or G-Sepharose FF column with adjusted buffer can be used for purification. The non-specifically bound components are removed by washing. Then the bound antibody was eluted by pH gradient method, and the antibody fragment was detected by SDS-PAGE and collected. The antibody can be filtered and concentrated by conventional methods. Soluble mixtures and polymers can also be removed by conventional methods, such as molecular sieves and ion exchange. The resulting product needs to be frozen immediately, such as at -70°C, or lyophilized.

The "treatment" used in the present disclosure refers to administering an internal or external therapeutic agent, such as a composition containing any one of the binding compounds of the present invention, to a patient who has one or more disease symptoms, and the therapeutic agent is known to have a therapeutic effect on these symptoms.

Humans and animals have quite different tolerance to the same medicament. Generally speaking, animals are more tolerant than humans. Generally, the following ratios are used to perform conversion: the dosage for human is set as 1, 25-50 for mice and rats, 15-20 for rabbits and guinea pigs, and 5-10 for dogs and cats. In addition, human and animal surface area calculation methods can be used to perform conversion. 1) Human surface area calculation methods are generally considered, such as Xu Wen's formula (Chinese Journal of Physiology, 12, 327, 1937) and Mech-Rubner's formula. The above method can be applied to the conversion of medicament dosage between human and different kinds of animals in the present disclosure.

The "homology" used in the present disclosure refers to the sequence similarity between two polynucleotide sequences or between two polypeptides. When the positions in the two sequences to be compared are occupied by the same base or amino acid monomer subunit, for example, each position of the two DNA molecules is occupied by adenine, then the molecules are deemed as homologous at that position. The percentage of homology between two sequences is a function of the number of matching or homologous positions shared by two sequences divided by the number of positions to be compared $\times$ 100. For example, in an optimal sequence alignment, when there are 6 matched or homologous positions among 10 positions in two sequences, then the two sequences are deemed as 60% homology; when there are 95 matched or homologous positions among 100 positions in two sequences, then the two sequences are deemed as 95% homology. Generally speaking, the comparison is performed when two sequences are aligned to obtain the maximum percent homology.

The "pharmaceutical composition" used in the present disclosure refers to a mixture containing one or more of the compounds described herein or physiologically/pharmaceutically acceptable salts or precursor thereof and other chemical components. For example, the other components are physiological/pharmaceutically acceptable carriers and excipients. The purpose of the pharmaceutical composition is to promote the administration to the organism, which contributes to the absorption of the active ingredients and thereby the biological activity.

Overall survival (OS) refers to the duration from random period to the time of death due to any cause. For subject who is still alive at the last follow-up, the OS is counted as censored data at the time of the last follow-up. For subject who is lost to follow-up, the OS is counted as censored data at the time of the last confirmed survival before being lost to follow-up. The OS with censored data is defined as the duration from random grouping to censoring.

Objective response rate (ORR) refers to the rate of patients whose tumors have shrunk to a certain level and maintained for a certain period of time, including CR and PR cases. The Response Evaluation Criteria in Solid Tumors (RECIST 1.1 Criteria) was used to assess the objective tumor response. Subjects must be accompanied with measurable tumor lesions at baseline, and the efficacy evaluation criteria are divided into complete remission (CR), partial remission (PR), stable disease (SD), and progressive disease (PD) according to the RECIST 1.1 criteria.

Disease Control Rate (DCR): The period starting from the time for first evaluation of the tumor as CR/PR/SD to the time for first evaluation as PD or death due to any cause.

12-month/24-month survival rate (Overall survival rate, OSR): The rate of cases that are still alive after 12-month/24-month follow-up since the first administration.

Disease Control Rate (DCR): refers to the rate of subjects with Best Overall Response (BOR) of complete remission (CR) or partial response (PR) or stable disease (SD≥8 weeks).

Complete Remission (CR): All target lesions disappear, and the short diameter of each of the all pathological lymph nodes (including target and non-target nodes) must be reduced to <10 mm.

Partial Remission (PR): The sum of diameters of all target lesions is reduced by at least 30% from the baseline level.

Progressive Disease (PD): The sum of diameters of all target lesions is increased by at least 20% compared to the reference, which is the minimum value of said sum measured during the entire experimental study (the baseline measurement value is set as the reference, if it is the minimum value); In addition, the absolute value of the sum of diameters must be increased by at least 5 mm (the presence of one or more new lesions is also deemed as progressive disease).

Stable Disease (SD): The degree of reduction for the target lesion does not reach PR, and the degree of increase does not reach PD level, which is somewhere in between. The minimum value of the sum of diameters can be used as a reference during the study.

**DESCRIPTION OF THE DRAWINGS**

Fig.1: The effect of TIM-3 antibodies on human non-small cell lung cancer HCC827 mice xenograft tumor.

Fig. 2: The effect of antibodies on the relative tumor volume in mice.

**DETAILED DESCRIPTION OF THE DISCLOSURE**

Hereinafter, the present disclosure is further described with reference to the examples. However, the scope of the present disclosure is not limited thereto.

**Example 1. Preparation of TIM-3 antigen and protein used for detection**

1. Design and expression of TIM-3 antigen

UniProt Hepatitis A virus cellular receptor 2 (human HAVCR2, human TIM-3, Uniprot No: Q8TDQ0) was used as the template of TIM-3 of the present invention, the amino acid sequence of the antigen and protein used for detection in the present invention were designed, optionally different tags were fused to the TIM-3 protein, and then cloned into pHr vector (house-made) or pTargeT vector (promega, A1410), respectively. The vectors were transiently expressed in 293 cells or stably expressed in CHO-S, and then purified to obtain the encoded antigen and protein used for detection in the present invention. Unless indicated specifically, the following TIM-3 antigens refer to human TIM-3.
Fusion protein of TIM-3 extracellular region and hIgG1 Fc: TIM-3-Fc (SEQ ID NO: 1), used for immunization of mouse:

MEFGLSWLFLVAILKGVQCSEVEYRAEVGQNAYLPCFYTPAAPGNLVPVCWGK
GACPVFECGNVVLRTDERDVNYWTSRYWLNGDFRKGDVSLTIENVTLADSGIY
CCRIQIPGIMNDEKFNLKLVIKPAKVTPAPTRQRDFTAAFPRMLTTRGHGPAETQT
LGSLPDINLTQISTLANELRDSRLANDLRDSGATIR*EPKSSDKTHTCPPCPAPELLG*
*GPSVFLFPPKPKDTLMISRTPEVTCVVVDVSHEDPEVKFNWYVDGVEVHNAKTKPR*
*EEQYNSTYRVVSVLTVLHQDWLNGKEYKCKVSNKALPAPIEKTISKAKGQPREPQVYT*
*LPPSRDELTKNQVSLTCLVKGFYPSDIAVEWESNGQPENNYKTTPPVLDSDGSFFLYS*
*KLTVDKSRWQQGNVFSCSVMHEALHNHYTQKSLSLSPGK;*

Note: The underlined part represents signal peptide, and the italicized part represents Fc.
TIM-3 Extracellular region with Flag and His tags: TIM-3-Flag-His (SEQ ID NO: 2), used for detection: TIM-3-flag-His

MEFGLSWLFLVAILKGVQCSEVEYRAEVGQNAYLPCFYTPAAPGNLVPVCWGK
GACPVFECGNVVLRTDERDVNYWTSRYWLNGDFRKGDVSLTIENVTLADSGIY
CCRIQIPGIMNDEKFNLKLVIKPAKVTPAPTRQRDFTAAFPRMLTTRGHGPAETQT
LGSLPDINLTQISTLANELRDSRLANDLRDSGATIRGSS*DYKDDDDKHHHHHH;*

Note: The underlined part represents signal peptide, and the italicized part represents Flag-His tag.
Full-length TIM-3: used to construct TIM-3-overexpressing cell lines:
TIM-3-full length (SEQ ID NO: 3)

<u>MFSHLPFDCVLLLLLLLLLTRS</u>SEVEYRAEVGQNAYLPCFYTPAAPGNLVPVCWG
KGACPVFECGNVVLRTDERDVNYWTSRYWLNGDFRKGDVSLTIENVTLADSGI
YCCRIQIPGIMNDEKFNLKLVIKPAKVTPAPTRQRDFTAAFPRMLTTRGHGPAET
QTLGSLPDINLTQISTLANELRDSRLANDLRDSGATIRIG<u>IYIGAGICAGLALALIF
GALIF</u>KWYSHSKEKIQNLSLISLANLPPSGLANAVAEGIRSEENIYTIEENVYEVEE
PNEYYCYVSSRQQPSQPLGCRFAMP;

Note: <u>Signal peptide</u> + extracellular region + <u>transmembrane region</u> + <u>intracellular region</u>.

2. Purification of TIM-3 related recombinant protein, and purification of hybridoma antibodies and recombinant antibodies

2.1 Purification steps of TIM-3-Flag-His recombinant protein:

The sample was centrifuged at high speed to remove impurities and concentrated to an appropriate volume. The NI-NTA affinity column (QIAGEN, Cat No. 30721) was equilibrated with PBS, and was washed with 2-5 times of column volume. After removing the impurities, the cell expression supernatant sample was loaded onto the column. The column was rinsed with PBS until the A280 reading dropped to the baseline. The column was rinsed with PBS to wash impurity proteins, and the target protein was collected. The target protein was eluted with washing buffer (20mM imidazole) and elution buffer (300mM imidazole) successively, and the elution peaks were collected.
The collected eluate was further purified by ion exchange (Hiload 16/600 Superdex 200 column). The column was equilibrated with about 2 column volumes of PBS to ensure pH 7.4. The elution buffer which has been identified to comprise the target protein was loaded after concentration, and the sample was collected, identifed by using SDS-PAGE and LC-MS, and was aliquoted for later use.

2.2 Purification of hybridomas, recombinant antibodies, and Fc fusion proteins

The cell expression supernatant sample was centrifuged at high speed to remove impurities, the hybridoma expression supernatant was purified by Protein G column, and the recombinant antibody and Fc fusion protein expression supernatant were purified by Protein A column. The column was rinsed with PBS until the A280 reading dropped to the baseline. The target protein was eluted with 100mM acetic acid pH3.0, and neutralized with 1M Tris-HCl pH8.0. The eluted sample was appropriately concentrated and further purified by PBS-equilibrated gel chromatography Superdex 200 (GE). The non-aggregate peaks were collected and aliquoted for later use.

**Example 2. Preparation of anti-human TIM-3 monoclonal antibody**

1. Animal immunization

Anti-human TIM-3 monoclonal antibody was produced by immunizing mice. SJL white mice, female, 6-8 weeks old (Beijing Charles River Laboratory Animal Technology Co., Ltd., animal production license number: SCXK (Beijing) 2012-0001) were used in the experiment. Feeding environment: SPF level. After the mice were purchased, they were adapted to the laboratory environment for 1 week, 12/12 hours light/dark cycle adjustment, temperature 20-25 °C; humidity 40-60%. Mice that have been adapted to the environment were immunized according to the following protocol. The antigen for immunization was the extracellular region of human TIM-3 with Fc-tag (SEQ ID NO: 1).
Immunization protocol: QuickAntibody-Mouse5W (KX0210041) was used to immunize mice. The ratio of antigen to adjuvant is 1:1, 10μg/mouse/time (first immunization/booster immunization). The antigen and adjuvant were quickly and thoroughly mixed and then inoculated. The inoculation period involved an interval of 21 days between the first and second immunizations, and an interval of 14 days between later immunizations. Blood was taken 7 days after each immunization, and the antibody titer in the mouse serum was determined by ELISA. The mice with high antibody titer in serum and with its titer reaching to the plateau were selected for splenocyte fusion. Three days before the fusion of splenocytes, the booster immunization was performed, and antigen solution prepared by physiological saline was injected at 20 μg/mouse by intraperitoneally (IP).

2. Splenocyte fusion

Optimized PEG-mediated fusion steps were used to fuse splenic lymphocytes with myeloma cells Sp2/0 cells (ATCC® CRL-8287™) to obtain hybridoma cells. The fused hybridoma cells were re-suspended in complete medium (DMEM medium comprising 20% FBS, 1×HAT, 1×OPI) at a density of 4-5 E5/ml, and were seeded onto a 96-well plate at 100 μl/well, and incubated at 37°C in 5% $CO_2$ for 3-4 days, and then HAT complete medium was added at 100μl/well to further cultivate the cells for 3-4 days until pinpoint-like clones were formed. The supernatant was removed, 200μl/well of HT complete medium (RPMI-1640 medium comprising 20% FBS, 1×HT and 1×OPI) was added, and incubated at 37°C in 5% $CO_2$ for 3 days, and then ELISA detection was performed.

## 3. Screening of hybridoma cell

According to the growth density of hybridoma cells, the hybridoma culture supernatant was detected by binding ELISA method (see Example 4, Test Example 1). TIM-3 overexpressing cell binding experiment was performed using cell supernatant in positive wells which were identified in the binding ELISA method (see Example 4, Test Example 2). The cells in wells that are positive for both protein-binding and cell-binding should be expanded in time for cryopreservation, and subcloned two to three times until single cell clone can be obtained.

TIM-3 binding ELISA and cell binding experiments were required for each subcloning of cells. The hybridoma clones were screened through the above experiments, and the secreted antibodies mAb-1701 and mAb-1799 were obtained. The antibodies were further prepared by the serum-free cell culture method. The antibodies were purified according to the purification example, and were provided for use in the test examples.

## 4. Sequencing of hybridoma positive clones

The process for cloning the sequences from the positive hybridoma was as follows. The hybridoma cells at logarithmic growth phase were collected, RNA was extracted by Trizol (Invitrogen, Cat No. 15596-018) according to the kit instructions, and PrimeScript™ Reverse Transcriptase kit was used for reverse transcription (Takara, Cat No. 2680A). The cDNA obtained by reverse transcription was amplified by PCR using mouse Ig-Primer Set (Novagen, TB326 Rev. B 0503) and was delivered to company for sequencing. The amino acid sequences corresponding to the DNA sequences for heavy chain and light chain variable region(s) of mAb-1701 and mAb-1799 were obtained:

mAb-1701 heavy chain variable region (SEQ ID NO: 4)

EVQLQQSGPELVKPGASVKISCKASGYTFT<u>DYYMN</u>WVKQSHGKSLEWIA<u>DIIPN NGGSKYNQKFKD</u>KATLTVDKSSSTAYMELRSLTSEDSAVYYCAT<u>WGYGSSYRW FDY</u>WGQGTLVSVSA;

mAb-1701 light chain variable region (SEQ ID NO: 5)

DIQMTQSPASQSASLGESVTITC<u>LASQPIGIWLA</u>WYQQKPGKSPQLLIY<u>AATSLA D</u>GVPSRFSGSGSGTKFSFKISSLQAEDFVSYYC<u>QQLYSSPWT</u>FGGGTKLEIK;

mAb-1799 heavy chain variable region (SEQ ID NO: 6)

EVKLVESEGGLVQPGSSMKLSCTASGFTFS<u>DYYMA</u>WVRQVPEKGLEWVA<u>NINY DGSSTYYLDSLKS</u>RFIISRDNAKNILYLQMNSLKSDDTATYYCAR<u>DVGYYGGNY GFAY</u>WGQGTLVTVSA;

mAb-1799 light chain variable region (SEQ ID NO: 7)

DIQMTQSPASLSASVGETVTITC<u>RASDNIYSYLA</u>WYQQKQGKSPQLLVY<u>NAKTL AE</u>GVPSRFSGSGSGTQFSLKINSLQPEDFGSYYC<u>QQHYGSPLT</u>FGAGTKLELK.

Wherein, the CDR sequences in the light and heavy chains of each antibody are shown in Table 1.

Table 1. Sequences of CDRs of each heavy chain and light chain

| Ab | Heavy chain | | Light chain | |
|---|---|---|---|---|
| 1701 | HCDR1 | DYYMN SEQ ID NO: 8 | LCDR1 | LASQPIGIWLA SEQ ID NO: 11 |
| | HCDR2 | DIIPNNGGSKYNQKFKD SEQ ID NO: 9 | LCDR2 | AATSLAD SEQ ID NO: 12 |
| | HCDR3 | WGYGSSYRWFDY SEQ ID NO: 10 | LCDR3 | QQLYSSPWT SEQ ID NO: 13 |
| 1799 | HCDR1 | DYYMA SEQ ID NO: 14 | LCDR1 | RASDNIYSYLA SEQ ID NO: 17 |
| | HCDR2 | NINYDGSSTYYLDSLKS SEQ ID NO: 15 | LCDR2 | NAKTLAE SEQ ID NO: 18 |
| | HCDR3 | DVGYYGGNYGFAY SEQ ID NO: 16 | LCDR3 | QQHYGSPLT SEQ ID NO: 19 |

**Example 3. Humanization of anti-human TIM-3 murine hybridoma monoclonal antibody**

1. Humanization of anti-TIM-3 antibody mAb-1701

By aligning against IMGT germline gene database of human antibody heavy and light chain variable region by MOE software, the heavy chain and light chain variable region germline genes with high homology to mAb-1701 antibody were selected as templates, and the CDRs of murine antibody were respectively grafted onto the corresponding human template to form the variable region in the order of FR1-CDR1-FR2-CDR2-FR3-CDR3-FR4. The amino acid residues are identified and annotated by Kabat numbering system.

1.1 Humanized framework selection for hybridoma clone mAb-1701

The light chain templates for humanization of the murine antibody mAb-1701 were IGKV1-33*01 and hjk4.1, and the heavy chain templates for humanization were IGHV1-18*01 and hjh4.1. The humanized variable region sequences are as follows:

h1701VH-CDR graft (SEQ ID NO: 20)

*QVQLVQSGAEVKKPGASVKVSCKASGYTFT*<u>DYYMN</u>*WVRQAPGQGLEWMG*<u>DIIPNN GGSKYNQKFKD</u>*RVTMTTDTSTSTAYMELRSLRSDDTAVYYCAR*<u>WGYGSSYRWFDY</u> *WGQGTLVTVSS;*

h1701VL-CDR graft (SEQ ID NO: 21)

*DIQMTQSPSSLSASVGDRVTITC*<u>LASQPIGIWLA</u>*WYQQKPGKAPKLLIY*<u>AATSLAD</u>*G VPSRFSGSGSGTDFTFTISSLQPEDIATYYC*<u>QQLYSSPWT</u>*FGGGTKVEIK;*

Note: The order is FR1-CDR1-FR2-CDR2-FR3-CDR3-FR4, the italics in the sequence represent FR sequence, and the underlined part represents CDR sequences.

1.2 Template selection and back-mutation(s) design for h1701

The specific mutation design is shown in Table 2 below:

Table 2. Template selection and back-mutation(s) design for h1701

| h1701_VL | | h1701_VH | |
|---|---|---|---|
| h1701_VL.1 | Grafted | h1701_VH.1 | Grafted |
| h1701_VL.1A | A43S | h1701_VH.1A | M48I |
| | | h1701_VH.1B | R98T |
| | | h1701_VH.1C | M48I, R98T |

(continued)

| h1701_VL | | h1701_VH | |
|---|---|---|---|
| | | h1701_VH.1D | M48I, R98T, R38K, D89E |
| | | h1701_VH.1E | M48I, R98T, G49A, V68A, M70L |
| | | h1701_VH.1F | M48I, R98T, G49A, V68A, M70L, R38K, D89E |
| Note: For example, A43S means that A at position 43 is mutated back to S, according to the Kabat numbering system. "Grafted" represents the sequence of murine antibody CDRs implanted into human germline FR region. | | | |

Table 3. Combination of h1701 humanized antibody heavy chain variable region and light chain variable region sequences

| | h1701_VL.1 | h1701_VL.1A |
|---|---|---|
| h1701_VH.1 | h1701-005 | h1701-006 |
| h1701_VH.1A | h1701-007 | h1701-008 |
| h1701_VH.1B | h1701-009 | h1701-010 |
| h1701_VH.1C | h1701-011 | h1701-012 |
| h1701_VH.1D | h1701-013 | h1701-014 |
| h1701_VH.1E | h1701-015 | h1701-016 |
| h1701_VH.1F | h1701-017 | h1701-018 |
| Note: This table shows the sequences resulted from various combinations of the mutations. As indicated by h1701-007, the humanized murine antibody h1701-007 has two mutants (light chain h1701_VL.1A and heavy chain h1701_VH. 1A). Others can be indicated in similar way. | | |

The particular sequence of humanized 1701 is as follows:

>h1701_VH.1 (the same as h1701VH-CDR graft, SEQ ID NO: 22)

QVQLVQSGAEVKKPGASVKVSCKASGYTFTDYYMNWVRQAPGQGLEWMGDII
PNNGGSKYNQKFKDRVTMTTDTSTSTAYMELRSLRSDDTAVYYCARWGYGSSY
RWFDYWGQGTLVTVSS;

>h1701h1701_VH.1A (SEQ ID NO: 23)

QVQLVQSGAEVKKPGASVKVSCKASGYTFTDYYMNWVRQAPGQGLEWIGDIIP
NNGGSKYNQKFKDRVTMTTDTSTSTAYMELRSLRSDDTAVYYCARWGYGSSYR
WFDYWGQGTLVTVSS;

>h1701_VH.1B (SEQ ID NO: 24)

QVQLVQSGAEVKKPGASVKVSCKASGYTFTDYYMNWVRQAPGQGLEWMGDII
PNNGGSKYNQKFKDRVTMTTDTSTSTAYMELRSLRSDDTAVYYCATWGYGSSY
RWFDYWGQGTLVTVSS;

>h1701_VH.1C (SEQ ID NO: 25)

QVQLVQSGAEVKKPGASVKVSCKASGYTFTDYYMNWVRQAPGQGLEWIGDIIP
NNGGSKYNQKFKDRVTMTTDTSTSTAYMELRSLRSDDTAVYYCATWGYGSSYR
WFDYWGQGTLVTVSS;

>h1701_VH.1D (SEQ ID NO: 26)

QVQLVQSGAEVKKPGASVKVSCKASGYTFTDYYMNWVKQAPGQGLEWIGDII
PNNGGSKYNQKFKDRVTMTTDTSTSTAYMELRSLRSEDTAVYYCATWGYGSSY
RWFDYWGQGTLVTVSS;

>h1701_VH.1E (SEQ ID NO: 27)

QVQLVQSGAEVKKPGASVKVSCKASGYTFTDYYMNWVRQAPGQGLEWIADIIP
NNGGSKYNQKFKDRATLTTDTSTSTAYMELRSLRSDDTAVYYCATWGYGSSYR
WFDYWGQGTLVTVSS;

>h1701_VH.1F (SEQ ID NO: 28)

QVQLVQSGAEVKKPGASVKVSCKASGYTFTDYYMNWVKQAPGQGLEWIADII
PNNGGSKYNQKFKDRATLTTDTSTSTAYMELRSLRSEDTAVYYCATWGYGSSYR
WFDYWGQGTLVTVSS;

>h1701_VL.1 (the same as h1701VL-CDR graft, SEQ ID NO: 29)

DIQMTQSPSSLSASVGDRVTITCLASQPIGIWLAWYQQKPGKAPKLLIYAATSLA
DGVPSRFSGSGSGTDFTFTISSLQPEDIATYYCQQLYSSPWTFGGGTKVEIK;

>h1701_VL.1A (SEQ ID NO: 30)

DIQMTQSPSSLSASVGDRVTITCLASQPIGIWLAWYQQKPGKSPKLLIYAATSLAD
GVPSRFSGSGSGTDFTFTISSLQPEDIATYYCQQLYSSPWTFGGGTKVEIK.

2. Humanization of anti-TIM-3 antibody mAb-1799

By aligning against IMGT germline gene database of human antibody heavy and light chain variable region by MOE software, the heavy chain and light chain variable region germline genes with high homology to mAb-1799 antibody were selected as templates, and the CDRs of murine antibody were respectively grafted onto the corresponding human template to form the variable region in the order of FR1-CDR1-FR2-CDR2-FR3-CDR3-FR4. The amino acid residues are identified and annotated by Kabat numbering system.

2.1 Humanized framework selection for hybridoma clone 1799

The light chain templates for humanization of the murine antibody 1799 were IGKV1-39*01 and hjk2.1, and the heavy chain templates for humanization were IGHV3-7*01 and hjh4.1. The humanized variable region sequences are as follows:

h1799VH-CDR graft (SEQ ID NO: 31)

*EVQLVESGGGLVQPGGSLRLSCAASGFTFS<u>DYYMA</u>WVRQAPGKGLEWVA<u>NINYDG</u>*

*SSTYYLDSLKSRFTISRDNAKNSLYLQMNSLRAEDTAVYYCARDVGYYGGNYGFAY*
*WGQGTLVTVSS;*

h1799VL-CDR graft (SEQ ID NO: 32)

*DIQMTQSPSSLSASVGDRVTITCRASDNIYSYLAWYQQKPGKAPKLLIYNAKTLAEG*
*VPSRFSGSGSGTDFTLTISSLQPEDFATYYCQQHYGSPLTFGQGTKLEIK;*

Note: The order is FR1-CDR1-FR2-CDR2-FR3-CDR3-FR4, the italics in the sequence represent FR sequence, and the underlined part represents CDR sequences.

2.2 Template selection and back-mutation(s) design of hybridoma clone 1799, see Table 4 below:

Table 4. Template selection and back-mutation(s) design of h1799

| h1799_VL | | h1799_VH | |
|---|---|---|---|
| h1799_VL.1 | Grafted | h1799_VH.1 | Grafted |
| h1799_VL.1A | I48V | h1799_VH.1A | Q3K |
| h1799_VL.1B | I48V, K45Q | h1799_VH.1B | Q3K, R87K |
| h1799_VL.1C | I48V, K45Q, A43S | | |
| h1799_VL.1D | I48V, K45Q, A43S, T85S | | |
| Note: For example, I48V means that I at position 48 is mutated back to V, according to the Kabat numbering system. "Grafted" represents the sequence of murine antibody CDRs implanted into human germline FR region. | | | |

Table 5. Combination of humanized antibody heavy chain variable region and light chain variable region sequences, for murine antibody 1799

| | h1799_VL.1 | h1799_VL.1A | h1799_VL.1B | h1799_VL.1C | h1799_VL.1D |
|---|---|---|---|---|---|
| h1799_VH.1 | h1799-005 | h1799-006 | h1799-007 | h1799-008 | h1799-009 |
| h1799_VH.1A | h1799-010 | h1799-011 | h1799-012 | h1799-013 | h1799-014 |
| h1799_VH.1B | h1799-015 | h1799-016 | h1799-017 | h1799-018 | h1799-019 |
| Note: This table shows the sequences resulted from various combinations of the mutations. As indicated by h1799-005, the humanized murine antibody h1799-005 has two mutants (light chain h1799_VL.1A and heavy chain h1799_VH. 1). Others can be indicated in similar way. | | | | | |

The particular sequence of the humanized 1799 is as follows:

>h1799_VH.1 (the same as h1799VH-CDR graft, SEQ ID NO: 33)

EVQLVESGGGLVQPGGSLRLSCAASGFTFSDYYMAWVRQAPGKGLEWVANINY
DGSSTYYLDSLKSRFTISRDNAKNSLYLQMNSLRAEDTAVYYCARDVGYYGGN
YGFAYWGQGTLVTVSS;

>h1799_VH.1A (SEQ ID NO: 34)

EVKLVESGGGLVQPGGSLRLSCAASGFTFSDYYMAWVRQAPGKGLEWVANINY
DGSSTYYLDSLKSRFTISRDNAKNSLYLQMNSLRAEDTAVYYCARDVGYYGGN

YGFAYWGQGTLVTVSS;

>h1799_VH.1B (SEQ ID NO: 35)

EVKLVESGGGLVQPGGSLRLSCAASGFTFSDYYMAWVRQAPGKGLEWVANINY
DGSSTYYLDSLKSRFTISRDNAKNSLYLQMNSLKAEDTAVYYCARDVGYYGGN
YGFAYWGQGTLVTVSS;

>h1799_VL.1 (the same as h1799VL-CDR graft, SEQ ID NO: 36)

DIQMTQSPSSLSASVGDRVTITCRASDNIYSYLAWYQQKPGKAPKLLIYNAKTL
AEGVPSRFSGSGSGTDFTLTISSLQPEDFATYYCQQHYGSPLTFGQGTKLEIK;

>h1799_VL.1A (SEQ ID NO: 37)

DIQMTQSPSSLSASVGDRVTITCRASDNIYSYLAWYQQKPGKAPKLLVYNAKTL
AEGVPSRFSGSGSGTDFTLTISSLQPEDFATYYCQQHYGSPLTFGQGTKLEIK;

>h1799_VL.1B (SEQ ID NO: 38)

DIQMTQSPSSLSASVGDRVTITCRASDNIYSYLAWYQQKPGKAPQLLVYNAKTL
AEGVPSRFSGSGSGTDFTLTISSLQPEDFATYYCQQHYGSPLTFGQGTKLEIK;

>h1799_VL.1C (SEQ ID NO: 39)

DIQMTQSPSSLSASVGDRVTITCRASDNIYSYLAWYQQKPGKSPQLLVYNAKTL
AEGVPSRFSGSGSGTDFTLTISSLQPEDFATYYCQQHYGSPLTFGQGTKLEIK;

>h1799_VL.1D (SEQ ID NO: 40)

DIQMTQSPSSLSASVGDRVTITCRASDNIYSYLAWYQQKPGKSPQLLVYNAKTL
AEGVPSRFSGSGSGTDFTLTISSLQPEDFASYYCQQHYGSPLTFGQGTKLEIK.

**Example 4. Preparation and effect test of recombinant chimeric antibody and humanized antibody**

For the antibodies, the constant regions of human heavy chain IgG4/light chain kappa were combined with each of the corresponding variable regions, and S228P mutation was introduced in the Fc section to increase the stability of the IgG4 antibody. Other mutations known in the art can also be used to improve its performance.
The sequence of the heavy chain constant region is shown in SEQ ID NO: 41:

ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K;

The sequence of the light chain constant region is as shown in SEQ ID NO: 42:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC.

### 1. Molecular cloning of recombinant chimeric antibodies

The positive antibody molecules obtained from hybridoma screening were sequenced to obtain the sequence of variable region coding gene. The forward and reverse primers were designed based on the sequence obtained by sequencing, and the sequenced gene was served as template; various antibody VH/VK gene fragments were constructed by PCR, and then homologously recombined with expression vector pHr (with signal peptide and hIgG4/hkappa constant region gene (CH1-FC/CL) fragment), and recombinant chimeric antibody full-length expression plasmids VH-CH1-FC-pHr/VL-CL -pHr were constructed for the two chimeric antibodies Ch1701 and Ch1799.

### 2. Molecular cloning of humanized antibodies

The antibody sequences after humanization design were subjected to codon optimization to obtain the coding gene sequence having human codon preference, primers were designed to construct various antibody VH/VK gene fragments by PCR, and then the fragments were homologously recombined with expression vector pHr (with signal peptide and hIgG4/hkappa constant region gene (CH1-FC/CL) fragment) to construct humanized antibody full-length expression plasmid VH-CH1-FC-pHr/VL-CL-pHr.

### 3. Expression and purification of recombinant chimeric antibodies and humanized antibodies

The plasmids separately expressing antibody light chain and heavy chain were transfected into HEK293E cells at a ratio of 1:1.2; the expression supernatant was collected 6 days later and centrifuged at high speed to remove impurities; and was purified with Protein A column. The column was rinsed with PBS until the A280 reading dropped to the baseline. The target protein was eluted with acidic elution solution, pH 3.0-pH 3.5, and was neutralized with 1M Tris-HCl pH 8.0-9.0. The eluted sample was appropriately concentrated and further purified by PBS-equilibrated gel chromatography Superdex 200 (GE). The aggregate peaks were removed, and the monomer peaks were collected and aliquoted for later use.

### Example 5. Site-directed mutation of h1701 antibody

Deamidation modification is a common chemical modification in antibodies that may affect the stability at later stage. Particularly, some amino acids in the CDR region(s) are highly deamidated, oxidized or isomerized; generally such mutations should be avoided or reduced as much as possible. According to accelerated stability experiments and computer-simulated antibody structure as well as hotspot prediction, the NNG in the heavy chain CDR2 of the h1701 antibody is the site susceptible to deamidation. The NNG described above are located at positions 54-56 in the heavy chain variable region of h1701 antibody respectively. According to properties of amino acids and technology for computer-simulated antibody structure, the amino acids at the above positions can be replaced with any amino acid. Preferably, the CDR2 mutant of h1701 is shown as: DIIP$X_1X_2X_3$GSKYNQKFKD (SEQ ID NO: 43), where $X_1$, $X_2$ and $X_3$ are amino acid residues at positions 54-56 in h1701 antibody heavy chain variable region; $X_1$ is selected from the group consisting of Asn, Leu, Val, Met and Glu; $X_2$ is selected from the group consisting of Asn, Glu, Met, His, Lys, Leu, Ala and Val; and $X_3$ is selected from the group consisting of Gly and Ala.

Further, the CDR2 comprising mutations at positions 54-56 described above can combine with the FR region comprising different back-mutation(s) to form the following heavy chain variable regions:

>h1701_VH.1-CDR2 mutant (SEQ ID NO: 44)

QVQLVQSGAEVKKPGASVKVSCKASGYTFTDYYMNWVRQAPGQGLEWMGDII
P$X_1X_2X_3$GSKYNQKFKDRVTMTTDTSTSTAYMELRSLRSDDTAVYYCARWGYGS
SYRWFDYWGQGTLVTVSS;

>h1701_VH.1A-CDR2 mutant (SEQ ID NO: 45)

QVQLVQSGAEVKKPGASVKVSCKASGYTFTDYYMNWVRQAPGQGLEWIGDIIP$X_1X_2X_3$GSKYNQKFKDRVTMTTDTSTSTAYMELRSLRSDDTAVYYCARWGYGSSYRWFDYWGQGTLVTVSS;

>h1701_VH.1B-CDR2 mutant (SEQ ID NO: 46)

QVQLVQSGAEVKKPGASVKVSCKASGYTFTDYYMNWVRQAPGQGLEWMGDIIP$X_1X_2X_3$GSKYNQKFKDRVTMTTDTSTSTAYMELRSLRSDDTAVYYCATWGYGSSYRWFDYWGQGTLVTVSS;

>h1701_VH.1C-CDR2 mutant (SEQ ID NO: 47)

QVQLVQSGAEVKKPGASVKVSCKASGYTFTDYYMNWVRQAPGQGLEWIGDIIP$X_1X_2X_3$GSKYNQKFKDRVTMTTDTSTSTAYMELRSLRSDDTAVYYCATWGYGSSYRWFDYWGQGTLVTVSS;

>h1701_VH.1D-CDR2 mutant (SEQ ID NO: 48)

QVQLVQSGAEVKKPGASVKVSCKASGYTFTDYYMNWVKQAPGQGLEWIGDIIP$X_1X_2X_3$GSKYNQKFKDRVTMTTDTSTSTAYMELRSLRSEDTAVYYCATWGYGSSYRWFDYWGQGTLVTVSS;

>h1701_VH.1E-CDR2 mutant (SEQ ID NO: 49)

QVQLVQSGAEVKKPGASVKVSCKASGYTFTDYYMNWVRQAPGQGLEWIADIIP$X_1X_2X_3$GSKYNQKFKDRATLTTDTSTSTAYMELRSLRSDDTAVYYCATWGYGSSYRWFDYWGQGTLVTVSS;

>h1701_VH.1F-CDR2 mutant (SEQ ID NO: 50)

QVQLVQSGAEVKKPGASVKVSCKASGYTFTDYYMNWVKQAPGQGLEWIADIIP$X_1X_2X_3$GSKYNQKFKDRATLTTDTSTSTAYMELRSLRSEDTAVYYCATWGYGSSYRWFDYWGQGTLVTVSS.

Exemplary sequences related to the HCDR2 mutants of h1701 and the humanized sequence h1701_VH.1B-CDR2 mutant (SEQ ID NO: 46) comprising the corresponding CDR2 mutant are shown in the following mutants and Table 6.

As an example, the NNG in HCDR2 of h1701-009 was designed to be mutated as NLG, NVG, NNA, NMA, NEA, NHA, NMG, NEG, NKG, NAG or NHG (the sequences of the above heavy chain variable region CDR2 amino acid mutants are as shown in SEQ ID NOs: 51-61 respectively). The expression plasmid construction and 293E expression were carried out by method of molecular cloning, and the mutant antibodies were purified and then further tested for the affinity and stability.

The affinity detection results of the exemplary variants are shown in Test Examples 1 and 3 respectively.

A series of amino acid mutations were performed on h1701-009, the particularly related sequences include but not limited to those described in Table 6. The particular results of chemical stability test are shown in Test Example 9:

Table 6. Sequences of heavy chain variable region mutants of h1701-009 comprising anti-deamidation modification

| Heavy chain variable region | SEQ ID NO. for VH | Corresponding HCDR2 sequence |
|---|---|---|
| h1701-009 | SEQ ID NO: 24 | DIIPNNGGSKYNQKFKD (SEQ ID NO: 9) |
| h1701-009NLG | SEQ ID NO: 51 | DIIPNLGGSKYNQKFKD (SEQ ID NO: 62) |

(continued)

| Heavy chain variable region | SEQ ID NO. for VH | Corresponding HCDR2 sequence |
|---|---|---|
| h1701-009NVG | SEQ ID NO: 52 | DIIPNVGGSKYNQKFKD (SEQ ID NO: 63) |
| h1701-009NNA | SEQ ID NO: 53 | DIIPNNAGSKYNQKFKD (SEQ ID NO: 64) |
| h1701-009NMA | SEQ ID NO: 54 | DIIPNMAGSKYNQKFKD (SEQ ID NO: 65) |
| h1701-009NEA | SEQ ID NO: 55 | DIIPNEAGSKYNQKFKD (SEQ ID NO: 66) |
| h1701-009NHA | SEQ ID NO: 56 | DIIPNHAGSKYNQKFKD (SEQ ID NO: 67) |
| h1701-009NMG | SEQ ID NO: 57 | DIIPNMGGSKYNQKFKD (SEQ ID NO: 68) |
| h1701-009NEG | SEQ ID NO: 58 | DIIPNEGGSKYNQKFKD (SEQ ID NO: 69) |
| h1701-009NKG | SEQ ID NO: 59 | DIIPNKGGSKYNQKFKD (SEQ ID NO: 70) |
| h1701-009NAG | SEQ ID NO: 60 | DIIPNAGGSKYNQKFKD (SEQ ID NO: 71) |
| h1701-009NHG | SEQ ID NO: 61 | DIIPNHGGSKYNQKFKD (SEQ ID NO: 72) |

**Test Example 1: Evaluation and comparison of the therapeutic effect of TIM-3 antibodies on human non-small cell lung cancer subcutaneous xenograft in HCC827 mice**

Laboratory animals and breeding conditions

NOG female mice were purchased from Beijing Charles River Laboratory Animal Technology Co., Ltd., (Beijing China, Certificate number 11400700200456, license SCXK (Beijing) 2016-0006), 4-6 week-old at the time of purchase, weighed about 18g, kept at 5 mice/cage, with 12/12 hours light/dark cycle adjustment, constant temperature of $23 \pm 1°C$, humidity of 50% to 60%, and food and water *ad libitum.*

Antibodies to be tested:

C25-hIgG4 (WTRC25, US6114143), at a concentration of 5.39mg/ml, and deliver quantity was 37.73mg.
h1799-005, at concentration of 12.00mg/ml, and deliver quantity was 27mg.
MBG-453 (Novartis AG), at a concentration of 5.44mg/ml, and deliver quantity was 25mg.
h1701-009NLG, at a concentration of 6.30mg/ml, and deliver quantity was 24mg.

Preparation method: the antibodies above were diluted to a concentration of 2mg/ml with PBS using pyrogen-free pipette tip under aseptic condition, divided into total of 10 tubes, 1.2ml/tube, stored at 4°C; 1 tube was taken out for each injection.

PBMCs extraction

The PBMCs used in this experiment were extracted from fresh blood of two volunteers. The extraction method was as follows:

a) The venous blood was treated with heparin to prevent agglutination, and mixed with equal volume of PBS comprising 2% FBS;
b) 15ml of separation solution 1077 was aseptically transferred into a 50ml separation tube (inverting the tube gently to fully mix 1077 in advance);
c) 25ml of diluted blood was carefully added to 1077 in a centrifuge tube (at room temperature, added slowly to form an obvious layer between blood and 1077; without mixing the diluted blood with 1077);
d) The sample was centrifuged at 1200g for 10 minutes at room temperature. Red blood cells and multi-nucleated white blood cells were precipitated by centrifugation, and meanwhile a layer of mononuclear lymphocytes was formed above 1077. The plasma 4-6 cm above the lymphocytes was aspirated;
e) The lymphocyte layer and half of 1077 below the lymphocyte layer were aspirated and transferred to another centrifuge tube. An equal volume of PBS was added and centrifuged at 300g for 8 minutes at room temperature;

f) The cells were washed with PBS or RPMI-1640 medium, and re-suspended with RPMI-1640 medium comprising serum.

Experimental steps:

200μl of HCC827 cells (1×10^7cells/mouse) (comprising 50% matrigel) were inoculated subcutaneously at right ribs of NOG mice. 16 days later, animals carrying too large or too small tumors were excluded, mice with the average tumor volume of about 215mm^3 were randomly divided into 4 groups: irrelevant antibody C25 IgG4 10mpk, MBG-453 10mpk, h1799-005 10mpk and h1701-009NLG 10mpk, 10 mice in each group (Day0); during the experiment, one animal in group #60-008L 10mpk exhibited persistent weight loss after injection of PBMCs and died on Day19 (suspected suffering from GVHD). In fact, 9 animals were included. PBMCs freshly extracted from two volunteers were mixed at a ratio of 1:1, and the mixture was injected intraperitoneally into NOG mice at 5×10^6 cells/100μl, and each of the antibodies was also injected intraperitoneally, twice per week for 7 times in total (Table 1); the tumor volume and animal weight were monitored twice per week, the data was recorded. At the end of the experiment, the animals were euthanized, and the tumor was taken and weighed.

Data processing

Plotting and statistical analysis of all data were performed by using Excel and GraphPad Prism 5 software.

The tumor volume (V) was calculated according to the formula: $V = 1/2 \times a \times b^2$, wherein a and b represent length and width, respectively.

Relative tumor proliferation rate T/C (%) = $(T-T_0)/(C-C_0) \times 100$, wherein T and C represent the tumor volume of the treatment group and the control group at the end of the experiment; $T_0$ and $C_0$ represent the tumor volume at the beginning of the experiment.

Tumor inhibition rate TGI (%) = 1-T/C (%).

Table 7: The therapeutic effect of TIM-3 antibodies on human non-small cell lung cancer xenograft in HCC827 mice

| Group | Numbers of animals[a] | administra tion cycle | administra tion route | D0 | D21 | |
|---|---|---|---|---|---|---|
| | | | | Mean±SEM (mm$^3$) | Mean±SEM (mm$^3$) | TGI (%) |
| C25 IgG4 | 10(10) | BIW×7 | I.P. | 215.6±12.1 | 577.1±82.9 | - |
| MBG-453 | 10(10) | BIW×7 | I.P. | 215.0±11.9 | 294.0±77.1* | 78.15 |
| h1799-005 | 10(10) | BIW×7 | I.P. | 215.1±12.6 | 139.7±14.3* ** | 120.86 |
| h1701-009 NLG | 9(10) | BIW×7 | I.P. | 210.5±15.5 | 264.7±89.2* | 85.01 |
| D0: the time for the first administration; a: actual numbers (grouping numbers) **p<0.01, ***p<0.001 v.s. C25 IgG4-10mpk by two-way ANOVA, Bonferroni's post-hoc test. i.p.: intraperitoneal injection. | | | | | | |

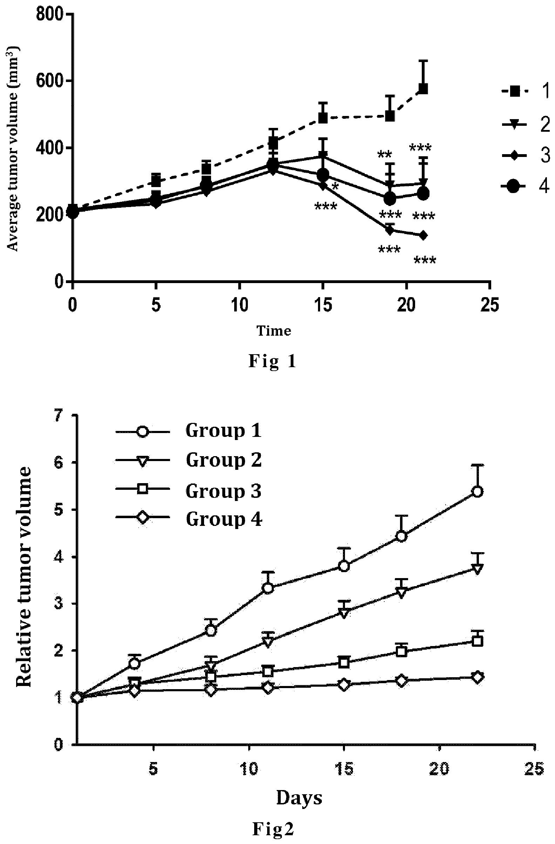

Experimental results show that: Three TIM-3 antibodies MBG-453 (10mpk, I.P., BIW×7), h1799-005 (10mpk, I.P., BIW×7) and h1701-009NLG (10mpk, I.P, BIW×7) can significantly inhibit the growth of human non-small cell lung cancer subcutaneous xenograft in HCC827 mice. On Day21 (the last measurement), the average volume of tumor in order from small to large is h1799-005 (10mpk, I.P, BIW×7), h1701-009NLG (10mpk, I.P., BIW×7) and MBG-453 (10mpk, I.P, BIW×7), respectively; and the tumor inhibition rates were 120.86% (p<0.001), 85.01% (p<0.05) and 78.15% (p<0.05) respectively (see Table 7 and Figure 1).

The tumor weights *in vitro* show tendency consistent with that observed for tumor volumes. The tumor weights of the three TIM-3 antibody groups were all significantly smaller than that of the irrelevant antibody C25 IgG4 (10mpk, I.P., BIW×7), and h1799-005 (10mpk, I.P., BIW×7), h1701-009NLG (10mpk, I.P., BIW×7) and MBG-453 (10mpk, I.P., BIW×7) exhibit the smallest, medium and the largest weights, respectively. All groups exhibited significant difference from C25 IgG4 (10mpk, I.P, BIW×7), p<0.001, p<0.05, and p<0.05 respectively.

Tumor-bearing mice were well tolerant to all TIM-3 antibodies, and only showed a slight change in the body weight during the whole administration process, no medicament-induced symptoms, such as obvious weight loss, were observed, except for one animal in h1701-009NLG (10mpk, I.P, BIW×7) group, which exhibited persistent weight loss after injection of PBMCs and was found dead on Day19 (the animal's abdomen was black when it was found dead, and the skin got rotten when being touched with tweezers, with obvious foul smell; the postmortem interval was estimated to be longer than 8 hours; considering the persistent weight loss before death, it was suspected suffering from GVHD due to being intolerant to the xenograft after transplantation of human PBMCs).

**Test Example 2: Evaluation and comparison of the effect of TIM-3 antibodies on mouse colon cancer MC38 subcutaneous xenograft**

Name of medicament to be tested:

TIM-3 antibody, h1799-005.
PD-1 antibody, murine PD-1 antibody J43 (J Immunol. 196(1):144-55.).

Experimental steps:

$1 \times 10^6$ mouse colon cancer MC38 cells were injected into the mouse's armpit. When the tumor was growing to an average volume of 50 to 200 mm$^3$, the animals were randomly divided into groups according to the tumor volume, and administered. 40 mice were divided into 4 groups: negative control group (group 1), TIM-3 antibody, 30 mg/kg group (group 2), PD-1 antibody, 5 mg/kg group (group 3), and TIM-3 antibody in combination with PD-1 antibody group (group 4), 10 animals in each group; each group was administered with corresponding concentration of test substance via tail vein injection at a dosing volume of 10ml/kg, and the dosing volume for the combined administration group was 20 mL/kg, twice per week, and administered for a period of 21 days.

Experimental results:

1. When compared with the tumor volume of 581 $\pm$ 63 mm$^3$ in negative control group, the tumor volumes for TIM-3 antibody 30 mg/kg group, PD-1 antibody 5 mg/kg group and combined administration group were 406 $\pm$ 31 (P<0.05), 245 $\pm$ 26 (*P*<0.01) and 166 $\pm$ 19 (*P*<0.001) mm$^3$, respectively, and significantly reduced;

2. When compared with the relative tumor volume (RTV) value of 5.38 $\pm$ 0.56 in negative control group, the RTV values for TIM-3 antibody 30 mg/kg group, PD-1 antibody 5 mg/kg group and combined administration group were 3.76 $\pm$ 0.32 (*P*<0.05), 2.20 $\pm$ 0.21 (*P*<0.01) and 1.44 $\pm$ 0.08 (*P*<0.001) respectively; T/C values were 69.91%, 40.92% and 26.66%, respectively;

3. When compared with the tumor weight of 0.3502 $\pm$ 0.0298g in negative control group, the tumor weight for TIM-3 antibody 30 mg/kg group, PD-1 antibody 5 mg/kg group and combined administration group were 0.2550 $\pm$ 0.0159 (*P*<0.01), 0.1820 $\pm$ 0.0178 (*P*<0.001) and 0.1102 $\pm$ 0.0106 g (*P*<0.001) respectively; IR were 27.19%, 48.05% and 65.36%, respectively;

4. The tumor volume, RTV value and tumor weight were analyzed. When compared with the TIM-3 antibody 30 mg/kg group, the combined administration group has significantly enhanced inhibition on tumor growth (P<0.001); when compared with the PD-1 antibody 5 mg/kg group, the combined administration group has significantly enhanced inhibition on tumor growth (P<0.05).

Table 8: The effect of antibodies on mouse colon cancer MC38 subcutaneous xenograft ($x \pm SE$)

| Group | dosage mg/kg | Average weight (g) | | Tumor volume (mm$^3$) | | RTV | T/C (%) |
|---|---|---|---|---|---|---|---|
| | | D1 | D22 | D1 | D22 | | |
| 1 | 30 | 30.0±0.5 | 31.0±0.5 | 112±9 | 581±63 | 5.38±0.56 | - |
| 2 | 30 | 29.9±0.6 | 30.8±0.5 | 112±8 | 406±31* | 3.76±0.32* | 69.91 |

(continued)

| Group | dosage mg/kg | Average weight (g) | | Tumor volume (mm$^3$) | | RTV | T/C (%) |
|---|---|---|---|---|---|---|---|
| | | D1 | D22 | D1 | D22 | | |
| 3 | 5 | 30.3±0.4 | 30.8±0.5 | 113±8 | 245±26** | 2.20±0.21** | 40.92 |
| 4 | 30+5 | 30.9±0.5 | 32.1±0.6 | 113±8 | 166±19***## #Δ | 1.44±0.08***### Δ | 26.66 |

When compared with Group 1, *: *P*<0.05, **: *P*<0.01, ***: *P*<0.001. When compared with Group 2, ###: *P*<0.001. When compared with Group 3, Δ: P<0.05.

Table 9: The effect of antibodies on tumor weight of mouse colon cancer MC38 subcutaneous xenograft ($x \pm SE$)

| Group | dosage mg/kg | Numbers of animals | | Tumor weight (g) | IR (%) |
|---|---|---|---|---|---|
| | | D1 | D22 | | |
| 1 | 30 | 10 | 10 | 0.3502±0.0298 | - |
| 2 | 30 | 10 | 10 | 0.2550±0.0159** | 27.19 |
| 3 | 5 | 10 | 10 | 0.1820±0.0.178*** | 48.05 |
| 4 | 30+5 | 10 | 10 | 0.1102±0.0106***###Δ | 65.36 |

When compared with Group 1, *: *P*<0.05, **: *P*<0.01, ***: *P*<0.001. When compared with Group 2, ###: *P*<0.001. When compared with Group 3, Δ: P<0.05, ΔΔ: *P*<0.01, ΔΔΔ: *P*<0.001.

## Claims

1. Use of a TIM-3 antibody or antigen-binding fragment thereof in the preparation of a medicament for treating tumor.

2. The use according to claim 1, wherein the TIM-3 antibody or antigen-binding fragment thereof comprises one or more CDR region sequences selected from the group consisting of:

    antibody heavy chain variable region HCDR sequences as shown in amino acid sequence SEQ ID NOs: 14, 15 and 16, or amino acid sequences having at least 95% sequence identity thereto; and
    antibody light chain variable region LCDR sequences as shown in amino acid sequence SEQ ID NOs: 17, 18 and 19, or amino acid sequences having at least 95% sequence identity thereto.

3. The use according to claim 2, wherein the TIM-3 antibody or antigen-binding fragment thereof is selected from the group consisting of murine antibody, chimeric antibody, humanized antibody or antigen-binding fragment thereof.

4. The use according to claim 3, wherein the humanized antibody comprises light chain FR region and heavy chain FR region sequences derived from human germline light chain and heavy chain or mutant sequences thereof, respectively.

5. The use according to claim 3, wherein the humanized antibody comprises heavy chain variable region as shown in SEQ ID NO: 31 or variant thereof, and preferably the variant comprises 1 to 10 amino acid alternations when compared with heavy chain variable region as shown in SEQ ID NO: 31, more preferably the amino acid alternations are amino acid back-mutations of Q3K and R87K; and the humanized antibody comprises light chain variable region as shown in SEQ ID NO: 32 or variant thereof, and preferably the variant comprises 1 to 10 amino acid alternations when compared with light chain variable region as shown in SEQ ID NO: 32, more preferably the amino acid alternation(s) is selected from the group consisting of amino acid back-mutations of Q3K and I48V, K45Q, A43S and T85S.

6. The use according to claim 3, wherein the humanized antibody comprises a heavy chain variable region as shown in SEQ ID NO: 33 and a light chain variable region as shown in SEQ ID NO: 36.

7. The use according to any one of claims 1 to 6, wherein the TIM-3 antibody is a full-length antibody which further comprises human antibody constant region(s), preferably comprises human heavy chain constant region as shown in SEQ ID NO: 41 and preferably comprises human light chain constant region as shown in SEQ ID NO:42.

8. The use according to any one of claims 1 to 6, wherein the antigen-binding fragment is selected from the group consisting of Fab, Fab', F(ab')2, single-chain antibody (scFv), dimerized V region (diabody), disulfide bond stabilized V region (dsFv), and antigen-binding fragment of peptide containing CDRs.

9. The use according to any one of claims 1 to 8, wherein the tumor is selected from the group consisting of breast cancer, lung cancer, liver cancer, gastric cancer, colorectal cancer, kidney cancer, melanoma and non-small cell lung cancer; preferably selected from the group consisting of non-small cell lung cancer, breast cancer, melanoma, liver cancer, colorectal cancer and kidney cancer; more preferably colorectal cancer or non-small cell lung cancer.

10. The use according to any one of claims 1 to 9, which is the use of the TIM-3 antibody or antigen-binding fragment thereof in combination with an anti-PD-1 antibody or antigen-binding fragment thereof for the preparation of a medicament for treating tumor.

11. The use according to claim 10, wherein the anti-PD-1 antibody or antigen-binding fragment thereof is humanized antibody or fragment thereof.

12. The use according to claim 10 or 11, wherein the antigen-binding fragment is selected from the group consisting of Fab, Fab'-SH, Fv, scFv and (Fab')$_2$ fragment.

13. The use according to claim 11 or 12, wherein the anti-PD-1 antibody or antigen-binding fragment thereof comprises heavy chain constant region of human IgG1, IgG2, IgG3 or IgG4 isotype, preferably heavy chain constant region of IgG1 or IgG4 isotype.

14. The use according to claim 13, wherein the anti-PD-1 antibody or antigen-binding fragment thereof comprises light chain constant region of kappa or lambda.

15. The use according to claim 11, wherein the anti-PD-1 antibody comprises a light chain variable region as shown in SEQ ID NO: 82 or variant thereof, and preferably the variant has 0-10 amino acid alternation(s) in the light chain variable region, more preferably the amino acid alternation is A43S; and the anti-PD-1 antibody comprises a heavy chain variable region as shown in SEQ ID NO: 81 or variant thereof, and preferably the variant has 0-10 amino acid alternation(s) in the heavy chain variable region, more preferably the amino acid alternation is G44R.

16. The use according to claim 11, wherein the anti-PD-1 antibody comprises a light chain as shown in SEQ ID NO: 80 or variant thereof, and preferably the variant has 0-10 amino acid alternation(s) in the light chain variable region, more preferably the amino acid alternation is A43S; and the anti-PD-1 antibody comprises a heavy chain as shown in SEQ ID NO: 79 or variant thereof, and preferably the variant has 0-10 amino acid alternation(s) in the heavy chain variable region, more preferably the amino acid alternation is G44R.

17. The use of claim 11, wherein the anti-PD-1 antibody comprises a light chain as shown in SEQ ID NO: 80 and a heavy chain as shown in SEQ ID NO: 79.

18. The use of claim 1, wherein the TIM-3 antibody or antigen-binding fragment thereof is administered in a human subject at a dosage ranging from 0.1 mg/kg to 10.0 mg/kg.

19. The use according to claim 10, wherein the anti-PD-1 antibody or antigen-binding fragment thereof is administered in a human subject at a dosage ranging from 0.1 mg/kg to 20.0 mg/kg.

20. A pharmaceutical composition, comprising an anti-TIM-3 antibody or antigen-binding fragment thereof, and an anti-PD-1 antibody or antigen-binding fragment thereof.

Fig 1

Fig 2

<div align="center">**INTERNATIONAL SEARCH REPORT**</div>

| International application No. |
|---|
| **PCT/CN2019/101552** |

**A. CLASSIFICATION OF SUBJECT MATTER**

A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CPRSABS, SIPOABS, DWPI, CNTXT, WOTXT, EPTXT, USTXT, CNKI, 百度学术搜索, BAIDU XUESHU SEARCH, WEB OF SCIENCE, PubMed: T细胞免疫球蛋白黏蛋白分子3, 甲型肝炎病毒细胞受体2, TIM-3, HAVCR-2, PD-1 Genbank, EMBL: 基于SEQ ID NOs:31-33, 36, 41, 42, 79-82的检索, Search according to SEQ ID NOs: 31-33, 36, 41, 42, 79-82

**C. DOCUMENTS CONSIDERED TO BE RELEVANT**

| Category* | Citation of document, with indication, where appropriate, of the relevant passages | Relevant to claim No. |
|---|---|---|
| X | WO 2017079115 A1 (JANSSEN BIOTECH, INC.) 11 May 2017 (2017-05-11) see claims 1-36 | 1, 8-14, 18-20 |
| A | WO 2017079115 A1 (JANSSEN BIOTECH, INC.) 11 May 2017 (2017-05-11) see claims 1-36 | 2-7, 15-17 |
| X | WO 2018036561 A1 (BEIGENE, LTD. et al.) 01 March 2018 (2018-03-01) see claims 1-33 | 1, 8-14, 18-20 |
| A | WO 2018036561 A1 (BEIGENE, LTD. et al.) 01 March 2018 (2018-03-01) see claims 1-33 | 2-7, 15-17 |
| X | WO 2018106588 A1 (ELI LILLY AND COMPANY et al.) 14 June 2018 (2018-06-14) see claims 1-36 | 1, 8-14, 18-20 |
| A | WO 2018106588 A1 (ELI LILLY AND COMPANY et al.) 14 June 2018 (2018-06-14) see claims 1-36 | 2-7, 15-17 |
| X | US 2017368168 A1 (AGENUS INC.) 28 December 2017 (2017-12-28) see claims 1-112, and description, paragraph [0137] | 1, 8-14, 18-20 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **10 November 2019** | **28 November 2019** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2019/101552**

**C.    DOCUMENTS CONSIDERED TO BE RELEVANT**

| Category* | Citation of document, with indication, where appropriate, of the relevant passages | Relevant to claim No. |
|---|---|---|
| A | US 2017368168 A1 (AGENUS INC.) 28 December 2017 (2017-12-28)<br>see claims 1-112, and description, paragraph [0137] | 2-7, 15-17 |

Form PCT/ISA/210 (second sheet) (January 2015)

**INTERNATIONAL SEARCH REPORT**
Information on patent family members

International application No.

**PCT/CN2019/101552**

| Patent document cited in search report | | | Publication date (day/month/year) | Patent family member(s) | | | Publication date (day/month/year) |
|---|---|---|---|---|---|---|---|
| WO | 2017079115 | A1 | 11 May 2017 | BR | 112018008891 | A8 | 26 February 2019 |
| | | | | CN | 108697791 | A | 23 October 2018 |
| | | | | AU | 2016350700 | A1 | 17 May 2018 |
| | | | | EP | 3371226 | A4 | 17 July 2019 |
| | | | | WO | 2017079112 | A1 | 11 May 2017 |
| | | | | EA | 201891093 | A1 | 31 October 2018 |
| | | | | EP | 3370768 | A1 | 12 September 2018 |
| | | | | KR | 20180069070 | A | 22 June 2018 |
| | | | | JP | 2019500892 | A | 17 January 2019 |
| | | | | TW | 201730213 | A | 01 September 2017 |
| | | | | CN | 108473584 | A | 31 August 2018 |
| | | | | EP | 3370769 | A1 | 12 September 2018 |
| | | | | BR | 112018008867 | A2 | 06 November 2018 |
| | | | | CA | 3004134 | A1 | 11 May 2017 |
| | | | | KR | 20180072821 | A | 29 June 2018 |
| | | | | EP | 3370768 | A4 | 24 July 2019 |
| | | | | IL | 258909 | D0 | 28 June 2018 |
| | | | | CA | 3004117 | A1 | 11 May 2017 |
| | | | | SV | 2018005684 | A | 25 September 2018 |
| | | | | PH | 12018500906 | A1 | 05 November 2018 |
| | | | | PE | 13262018 | A1 | 20 August 2018 |
| | | | | AU | 2016348388 | A1 | 17 May 2018 |
| | | | | AU | 2016348391 | A1 | 17 May 2018 |
| | | | | EP | 3370769 | A4 | 22 May 2019 |
| | | | | US | 2017121409 | A1 | 04 May 2017 |
| | | | | BR | 112018008891 | A2 | 06 November 2018 |
| | | | | CN | 108430509 | A | 21 August 2018 |
| | | | | CL | 2018001177 | A1 | 12 October 2018 |
| | | | | WO | 2017079116 | A3 | 20 July 2017 |
| | | | | JP | 2019500891 | A | 17 January 2019 |
| | | | | KR | 20180069071 | A | 22 June 2018 |
| | | | | BR | 112018008904 | A2 | 27 November 2018 |
| | | | | CA | 3004138 | A1 | 11 May 2017 |
| | | | | WO | 2017079116 | A2 | 11 May 2017 |
| | | | | MX | 2018005551 | A | 09 November 2018 |
| | | | | JP | 2019500893 | A | 17 January 2019 |
| | | | | SG | 11201803520P | A | 30 May 2018 |
| | | | | BR | 112018008867 | A8 | 26 February 2019 |
| | | | | CR | 20180234 | A | 11 September 2018 |
| | | | | EP | 3371226 | A2 | 12 September 2018 |
| | | | | CO | 2018005614 | A2 | 31 May 2018 |
| | | | | AR | 106583 | A1 | 31 January 2018 |
| WO | 2018036561 | A1 | 01 March 2018 | EP | 3504243 | A1 | 03 July 2019 |
| | | | | US | 2019276533 | A1 | 12 September 2019 |
| | | | | BR | 112019003976 | A2 | 28 May 2019 |
| | | | | KR | 20190042037 | A | 23 April 2019 |
| | | | | TW | 201819414 | A | 01 June 2018 |
| | | | | CA | 3034962 | A1 | 01 March 2018 |
| | | | | SG | 11201901548S | A | 28 March 2019 |
| | | | | EA | 201990594 | A1 | 30 August 2019 |

Form PCT/ISA/210 (patent family annex) (January 2015)

**INTERNATIONAL SEARCH REPORT**
Information on patent family members

International application No.

**PCT/CN2019/101552**

| Patent document cited in search report | | | Publication date (day/month/year) | Patent family member(s) | | | Publication date (day/month/year) |
|---|---|---|---|---|---|---|---|
| | | | | CN | 109790218 | A | 21 May 2019 |
| | | | | AU | 2017317227 | A1 | 11 April 2019 |
| WO | 2018106588 | A1 | 14 June 2018 | TW | I648065 | B | 21 January 2019 |
| | | | | AR | 110203 | A1 | 06 March 2019 |
| | | | | AU | 2017372709 | A1 | 16 May 2019 |
| | | | | KR | 20190076029 | A | 01 July 2019 |
| | | | | EP | 3551662 | A1 | 16 October 2019 |
| | | | | TW | 201831198 | A | 01 September 2018 |
| | | | | CO | 2019005846 | A2 | 19 June 2019 |
| | | | | IL | 267025 | D0 | 31 July 2019 |
| | | | | BR | 112019009617 | A2 | 13 August 2019 |
| | | | | CN | 110267988 | A | 20 September 2019 |
| | | | | CA | 3043763 | A1 | 14 June 2018 |
| | | | | CR | 20190266 | A | 22 August 2019 |
| | | | | US | 2019276534 | A1 | 12 September 2019 |
| US | 2017368168 | A1 | 28 December 2017 | CA | 3024508 | A1 | 30 November 2017 |
| | | | | PH | 12018502400 | A1 | 08 April 2019 |
| | | | | KR | 20190021224 | A | 05 March 2019 |
| | | | | WO | 2017205721 | A1 | 30 November 2017 |
| | | | | EP | 3464360 | A1 | 10 April 2019 |
| | | | | SG | 11201810023Q | A | 28 December 2018 |
| | | | | MX | 2018014387 | A | 14 March 2019 |
| | | | | JP | 2019520809 | A | 25 July 2019 |
| | | | | TW | 201803903 | A | 01 February 2018 |
| | | | | BR | 112018074463 | A2 | 06 March 2019 |
| | | | | CN | 109476751 | A | 15 March 2019 |
| | | | | AU | 2017271588 | A1 | 17 January 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011159877 A
- WO 2013006490 A
- WO 2015117002 A
- WO 2016144803 A
- WO 2016161270 A
- US 20150218274 A
- WO 2018153366 A
- US 6114143 A

**Non-patent literature cited in the description**

- **NGIOW.** *Cancer Res.,* 2011, vol. 71 (21), 1-5
- **GUO.** *Journal of Translational Medicine,* 2013, vol. 11, 215
- **NGIOW.** *Cancer Res.,* 2011, vol. 71 (21), 6567-6571
- *Nature Communication,* February 2016
- *Nature,* vol. 545, 60-65
- **KABAT, EA.** Sequences of Proteins of Immunological Interest. 1991
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **KABAT E.A. et al.** Sequences of proteins of immunological interest. NIH, 1991
- *Chinese Journal of Physiology,* 1937, vol. 12, 327
- *J Immunol.,* vol. 196 (1), 144-55